# EUROPEAN PATENT APPLICATION

(11) **EP 4 501 958 A1**
(43) Date of publication of application: **05.02.2025**
(21) Application number: 23781054.4
(22) Date of filing: 31.03.2023
(51) Int. Cl.: C07K 16/18, C12N 15/13, G01N 33/53, G01N 33/577

(54) **HRG MEASURING METHOD USING ANTI-HRG MONOCLONAL ANTIBODIES**

(30) Priority: 31.03.2022 JP 2022058771
(71) Applicant: Shionogi & Co., Ltd, Osaka-shi, Osaka 541-0045 (JP); National University Corporation Okayama University, Kita-ku, Okayama-shi, Okayama 700-8530 (JP)
(72) Inventor: UCHIUMI Takaoki, Osaka-shi, Osaka 541-0045 (JP); OTA Norio, Osaka-shi, Osaka 541-0045 (JP); INOUE Youko, Osaka-shi, Osaka 541-0045 (JP); NISHI Hiroshi, Osaka-shi, Osaka 541-0045 (JP); SHIMOSAKO Kenichi, Osaka-shi, Osaka 541-0045 (JP); NISHIBORI Masahiro, Okayama-shi, Okayama 700-8530 (JP); MORIMATSU Hiroshi, Okayama-shi, Okayama 700-8530 (JP)
(74) Representative: Vossius & Partner Patentanwälte Rechtsanwälte mbB
(86) International application number: PCT/JP2023/013531
(87) International publication number: WO 2023/191046

(57) **Abstract**

An object of the present invention is to provide a monoclonal antibody capable of specifically detecting and capturing HRG in a test sample, a set (combination) thereof, and a method for measuring HRG using the same. As a result of intensive studies, the present inventors have found an antibody capable of detecting and capturing HRG in a test sample with good sensitivity and specificity, a set thereof, and a method for measuring HRG using the same, thereby completing the present invention.

## Description

### [TECHNICAL FIELD]

The present invention relates to a monoclonal antibody against a histidine-rich glycoprotein (hereinafter, may be simply referred to as "HRG"), a set (combination) thereof, and a method for measuring HRG using the same.

### [BACKGROUND ART]

Histidine-rich glycoprotein is a plasma protein with a molecular weight of about 80 kDa that was identified by Heimburger et al (1972) in 1972. Histidine-rich glycoprotein is a high histidine-containing protein composed of a total of 507 amino acids, of which 66 residues are histidine, is synthesized primarily in the liver, and is present in human plasma at a concentration of about 100 to 150 pg/mL, which is considered to be very high. Histidine-rich glycoprotein is known to be involved in the regulation of coagulation fibrinolytic system and the control of angiogenesis (Non-patent Document 1).

It is known that histidine-rich glycoprotein in blood is significantly lower in septic patients when comparing between septic patients and healthy person (Patent Document 1).

Patent Document 1 discloses a method for measuring blood HRG using a monoclonal antibody against HRG as a capture antibody and a NiNTA-HRP probe for detecting HRG. However, the measurement by this method has a high background and low sensitivity, and in particular, the detection system is NiNTA, which is problematic in that an amino acid sequence containing a large number of histidines other than HRG is detected. In addition, although HRG detection kits are commercially available from Sino Biological, Inc. and RayBiotech, Inc., they are not sufficient in terms of background and detection sensitivity.

### [PRIOR ART REFERENCES]

### [Patent Document]

[Patent Document 1] WO2013/183494

### [Non-patent Document]

[Non-patent Document 1] Blood, Vol. 117, No. 7, 2093-2101 (2011)

### [SUMMARY OF THE INVENTION]

### [PROBLEMS TO BE SOLVED BY THE INVENTION]

An object of the present invention is to provide a monoclonal antibody capable of specifically detecting and capturing HRG in a test sample, a set (combination) thereof, and a method for measuring HRG using the same.

### [MEANS FOR SOLVING THE PROBLEM]

As a result of intensive studies, the present inventors have found an antibody capable of detecting and capturing HRG in a test sample with good sensitivity and specificity, a set thereof, and a method for measuring HRG using the same, thereby completing the present invention.

Specifically, the present invention relates to the followings.
(1) A monoclonal antibody or antibody fragment thereof against HRG, comprising a heavy chain variable region and a light chain variable region according to any one of the following 1) to 6):
   1) a heavy chain variable region comprising a CDR1 consisting of an amino acid sequence in which one to two amino acids may be deleted, substituted, inserted and/or added in the amino acid sequence of SEQ ID NO: 41,
      a CDR2 consisting of an amino acid sequence in which one to two amino acids may be deleted, substituted, inserted and/or added in the amino acid sequence of SEQ ID NO: 42, and
      a CDR3 consisting of an amino acid sequence in which one to two amino acids may be deleted, substituted, inserted and/or added in the amino acid sequence of SEQ ID NO: 43,
         and
      a light chain variable region comprising a CDR1 consisting of an amino acid sequence in which one to two amino acids may be deleted, substituted, inserted and/or added in the amino acid sequence of SEQ ID NO: 7,
      a CDR2 consisting of an amino acid sequence in which one to two amino acids may be deleted, substituted, inserted and/or added in the amino acid sequence of SEQ ID NO: 45, and
      a CDR3 consisting of an amino acid sequence in which one to two amino acids may be deleted, substituted, inserted and/or added in the amino acid sequence of SEQ ID NO: 9,
   2) a heavy chain variable region comprising a CDR1 consisting of an amino acid sequence in which one to two amino acids may be deleted, substituted, inserted and/or added in the amino acid sequence of SEQ ID NO: 33,
      a CDR2 consisting of an amino acid sequence in which one to two amino acids may be deleted, substituted, inserted and/or added in the amino acid sequence of SEQ ID NO: 34, and
      a CDR3 consisting of an amino acid sequence in which one to two amino acids may be deleted, substituted, inserted and/or added in the amino acid sequence of SEQ ID NO: 35,
         and
      a light chain variable region comprising a CDR1 consisting of an amino acid sequence in which one to two amino acids may be deleted, substituted, inserted and/or added in the amino acid sequence of SEQ ID NO: 37,
      a CDR2 consisting of an amino acid sequence in which one to two amino acids may be deleted, substituted, inserted and/or added in the amino acid sequence of SEQ ID NO: 38, and
      a CDR3 consisting of an amino acid sequence in which one to two amino acids may be deleted, substituted, inserted and/or added in the amino acid sequence of SEQ ID NO: 39,
   3) a heavy chain variable region comprising a CDR1 consisting of an amino acid sequence in which one to two amino acids may be deleted, substituted, inserted and/or added in the amino acid sequence of SEQ ID NO: 3,
      a CDR2 consisting of an amino acid sequence in which one to two amino acids may be deleted, substituted, inserted and/or added in the amino acid sequence of SEQ ID NO: 4, and
      a CDR3 consisting of an amino acid sequence in which one to two amino acids may be deleted, substituted, inserted and/or added in the amino acid sequence of SEQ ID NO: 5,
         and
      a light chain variable region comprising a CDR1 consisting of an amino acid sequence in which one to two amino acids may be deleted, substituted, inserted and/or added in the amino acid sequence of SEQ ID NO: 7,
      a CDR2 consisting of an amino acid sequence in which one to two amino acids may be deleted, substituted, inserted and/or added in the amino acid sequence of SEQ ID NO: 8, and
      a CDR3 consisting of an amino acid sequence in which one to two amino acids may be deleted, substituted, inserted and/or added in the amino acid sequence of SEQ ID NO: 9,
   4) a heavy chain variable region comprising a CDR1 consisting of an amino acid sequence in which one to two amino acids may be deleted, substituted, inserted and/or added in the amino acid sequence of SEQ ID NO: 11,
      a CDR2 consisting of an amino acid sequence in which one to two amino acids may be deleted, substituted, inserted and/or added in the amino acid sequence of SEQ ID NO: 12, and
      a CDR3 consisting of an amino acid sequence in which one to two amino acids may be deleted, substituted, inserted and/or added in the amino acid sequence of SEQ ID NO: 13,
         and
      a light chain variable region comprising a CDR1 consisting of an amino acid sequence in which one to two amino acids may be deleted, substituted, inserted and/or added in the amino acid sequence of SEQ ID NO: 15,
      a CDR2 consisting of an amino acid sequence in which one to two amino acids may be deleted, substituted, inserted and/or added in the amino acid sequence of SEQ ID NO: 16, and
      a CDR3 consisting of an amino acid sequence in which one to two amino acids may be deleted, substituted, inserted and/or added in the amino acid sequence of SEQ ID NO: 17,
   5) a heavy chain variable region comprising a CDR1 consisting of an amino acid sequence in which one to two amino acids may be deleted, substituted, inserted and/or added in the amino acid sequence of SEQ ID NO: 21,
      a CDR2 consisting of an amino acid sequence in which one to two amino acids may be deleted, substituted, inserted and/or added in the amino acid sequence of SEQ ID NO: 22, and
      a CDR3 consisting of an amino acid sequence in which one to two amino acids may be deleted, substituted, inserted and/or added in the amino acid sequence of SEQ ID NO: 23,
         and
      a light chain variable region comprising a CDR1 consisting of an amino acid sequence in which one to two amino acids may be deleted, substituted, inserted and/or added in the amino acid sequence of SEQ ID NO: 25,
      a CDR2 consisting of an amino acid sequence in which one to two amino acids may be deleted, substituted, inserted and/or added in the amino acid sequence of SEQ ID NO: 26, and
      a CDR3 consisting of an amino acid sequence in which one to two amino acids may be deleted, substituted, inserted and/or added in the amino acid sequence of SEQ ID NO: 27, and
   6) a heavy chain variable region comprising a CDR1 consisting of an amino acid sequence in which one to two amino acids may be deleted, substituted, inserted and/or added in the amino acid sequence of SEQ ID NO: 11,
      a CDR2 consisting of an amino acid sequence in which one to two amino acids may be deleted, substituted, inserted and/or added in the amino acid sequence of SEQ ID NO: 29, and
      a CDR3 consisting of an amino acid sequence in which one to two amino acids may be deleted, substituted, inserted and/or added in the amino acid sequence of SEQ ID NO: 13,
         and
      a light chain variable region comprising a CDR1 consisting of an amino acid sequence in which one to two amino acids may be deleted, substituted, inserted and/or added in the amino acid sequence of SEQ ID NO: 15,
      a CDR2 consisting of an amino acid sequence in which one to two amino acids may be deleted, substituted, inserted and/or added in the amino acid sequence of SEQ ID NO: 31, and
      a CDR3 consisting of an amino acid sequence in which one to two amino acids may be deleted, substituted, inserted and/or added in the amino acid sequence of SEQ ID NO: 17,
(2) The monoclonal antibody or antibody fragment thereof according to (1), comprising a heavy chain variable region and a light chain variable region according to any one of the following 1) to 7):
   1) a heavy chain variable region consisting of an amino acid sequence in which one to two amino acids may be deleted, substituted, inserted and/or added in the amino acid sequence of SEQ ID NO: 40, and
      a light chain variable region consisting of an amino acid sequence in which one to two amino acids may be deleted, substituted, inserted and/or added in the amino acid sequence of SEQ ID NO: 44,
   2) a heavy chain variable region consisting of an amino acid sequence in which one to two amino acids may be deleted, substituted, inserted and/or added in the amino acid sequence of SEQ ID NO: 32, and
      a light chain variable region consisting of an amino acid sequence in which one to two amino acids may be deleted, substituted, inserted and/or added in the amino acid sequence of SEQ ID NO: 36,
   3) a heavy chain variable region consisting of an amino acid sequence in which one to two amino acids may be deleted, substituted, inserted and/or added in the amino acid sequence of SEQ ID NO: 2, and
      a light chain variable region consisting of an amino acid sequence in which one to two amino acids may be deleted, substituted, inserted and/or added in the amino acid sequence of SEQ ID NO: 6,
   4) a heavy chain variable region consisting of an amino acid sequence in which one to two amino acids may be deleted, substituted, inserted and/or added in the amino acid sequence of SEQ ID NO: 10, and
      a light chain variable region consisting of an amino acid sequence in which one to two amino acids may be deleted, substituted, inserted and/or added in the amino acid sequence of SEQ ID NO: 14,
   5) a heavy chain variable region consisting of an amino acid sequence in which one to two amino acids may be deleted, substituted, inserted and/or added in the amino acid sequence of SEQ ID NO: 18, and
      a light chain variable region consisting of an amino acid sequence in which one to two amino acids may be deleted, substituted, inserted and/or added in the amino acid sequence of SEQ ID NO: 19,
   6) a heavy chain variable region consisting of an amino acid sequence in which one to two amino acids may be deleted, substituted, inserted and/or added in the amino acid sequence of SEQ ID NO: 20, and
      a light chain variable region consisting of an amino acid sequence in which one to two amino acids may be deleted, substituted, inserted and/or added in the amino acid sequence of SEQ ID NO: 24, and
   7) a heavy chain variable region consisting of an amino acid sequence in which one to two amino acids may be deleted, substituted, inserted and/or added in the amino acid sequence of SEQ ID NO: 28, and
      a light chain variable region consisting of an amino acid sequence in which one to two amino acids may be deleted, substituted, inserted and/or added in the amino acid sequence of SEQ ID NO: 30.
(3) A set of monoclonal antibodies or antibody fragments thereof against HRG, for use in measurement of HRG in a test sample, which is selected from the following 1) or 2):
   1) a capture antibody is a monoclonal antibody or antibody fragment thereof against HRG, comprising:
      a heavy chain variable region comprising a CDR1 consisting of an amino acid sequence in which one to two amino acids may be deleted, substituted, inserted and/or added in the amino acid sequence of SEQ ID NO: 41,
      a CDR2 consisting of an amino acid sequence in which one to two amino acids may be deleted, substituted, inserted and/or added in the amino acid sequence of SEQ ID NO: 42, and
      a CDR3 consisting of an amino acid sequence in which one to two amino acids may be deleted, substituted, inserted and/or added in the amino acid sequence of SEQ ID NO: 43,
         and
      a light chain variable region comprising a CDR1 consisting of an amino acid sequence in which one to two amino acids may be deleted, substituted, inserted and/or added in the amino acid sequence of SEQ ID NO: 7,
      a CDR2 consisting of an amino acid sequence in which one to two amino acids may be deleted, substituted, inserted and/or added in the amino acid sequence of SEQ ID NO: 45, and
      a CDR3 consisting of an amino acid sequence in which one to two amino acids may be deleted, substituted, inserted and/or added in the amino acid sequence of SEQ ID NO: 9, and
      a detection antibody is a monoclonal antibody or antibody fragment thereof against HRG, comprising a heavy chain variable region and a light chain variable region according to any one of the following (i) to (iv):
         (i) a heavy chain variable region comprising a CDR1 consisting of an amino acid sequence in which one to two amino acids may be deleted, substituted, inserted and/or added in the amino acid sequence of SEQ ID NO: 11,
            a CDR2 consisting of an amino acid sequence in which one to two amino acids may be deleted, substituted, inserted and/or added in the amino acid sequence of SEQ ID NO: 12, and
            a CDR3 consisting of an amino acid sequence in which one to two amino acids may be deleted, substituted, inserted and/or added in the amino acid sequence of SEQ ID NO: 13,
               and
            a light chain variable region comprising a CDR1 consisting of an amino acid sequence in which one to two amino acids may be deleted, substituted, inserted and/or added in the amino acid sequence of SEQ ID NO: 15,
            a CDR2 consisting of an amino acid sequence in which one to two amino acids may be deleted, substituted, inserted and/or added in the amino acid sequence of SEQ ID NO: 16, and
            a CDR3 consisting of an amino acid sequence in which one to two amino acids may be deleted, substituted, inserted and/or added in the amino acid sequence of SEQ ID NO: 17,
         (ii) a heavy chain variable region comprising a CDR1 consisting of an amino acid sequence in which one to two amino acids may be deleted, substituted, inserted and/or added in the amino acid sequence of SEQ ID NO: 21,
            a CDR2 consisting of an amino acid sequence in which one to two amino acids may be deleted, substituted, inserted and/or added in the amino acid sequence of SEQ ID NO: 22, and
            a CDR3 consisting of an amino acid sequence in which one to two amino acids may be deleted, substituted, inserted and/or added in the amino acid sequence of SEQ ID NO: 23,
               and
            a light chain variable region comprising a CDR1 consisting of an amino acid sequence in which one to two amino acids may be deleted, substituted, inserted and/or added in the amino acid sequence of SEQ ID NO: 25,
            a CDR2 consisting of an amino acid sequence in which one to two amino acids may be deleted, substituted, inserted and/or added in the amino acid sequence of SEQ ID NO: 26, and
            a CDR3 consisting of an amino acid sequence in which one to two amino acids may be deleted, substituted, inserted and/or added in the amino acid sequence of SEQ ID NO: 27,
         (iii) a heavy chain variable region comprising a CDR1 consisting of an amino acid sequence in which one to two amino acids may be deleted, substituted, inserted and/or added in the amino acid sequence of SEQ ID NO: 11,
            a CDR2 consisting of an amino acid sequence in which one to two amino acids may be deleted, substituted, inserted and/or added in the amino acid sequence of SEQ ID NO: 29, and
            a CDR3 consisting of an amino acid sequence in which one to two amino acids may be deleted, substituted, inserted and/or added in the amino acid sequence of SEQ ID NO: 13,
               and
            a light chain variable region comprising a CDR1 consisting of an amino acid sequence in which one to two amino acids may be deleted, substituted, inserted and/or added in the amino acid sequence of SEQ ID NO: 15,
            a CDR2 consisting of an amino acid sequence in which one to two amino acids may be deleted, substituted, inserted and/or added in the amino acid sequence of SEQ ID NO: 31, and
            a CDR3 consisting of an amino acid sequence in which one to two amino acids may be deleted, substituted, inserted and/or added in the amino acid sequence of SEQ ID NO: 17, and
         (iv) a heavy chain variable region comprising a CDR1 consisting of an amino acid sequence in which one to two amino acids may be deleted, substituted, inserted and/or added in the amino acid sequence of SEQ ID NO: 33,
            a CDR2 consisting of an amino acid sequence in which one to two amino acids may be deleted, substituted, inserted and/or added in the amino acid sequence of SEQ ID NO: 34, and
            a CDR3 consisting of an amino acid sequence in which one to two amino acids may be deleted, substituted, inserted and/or added in the amino acid sequence of SEQ ID NO: 35,
               and
            a light chain variable region comprising a CDR1 consisting of an amino acid sequence in which one to two amino acids may be deleted, substituted, inserted and/or added in the amino acid sequence of SEQ ID NO: 37,
            a CDR2 consisting of an amino acid sequence in which one to two amino acids may be deleted, substituted, inserted and/or added in the amino acid sequence of SEQ ID NO: 38, and
            a CDR3 consisting of an amino acid sequence in which one to two amino acids may be deleted, substituted, inserted and/or added in the amino acid sequence of SEQ ID NO: 39,
   2) a capture antibody is a monoclonal antibody or antibody fragment thereof against HRG, consisting of:
      a heavy chain variable region comprising a CDR1 consisting of an amino acid sequence in which one to two amino acids may be deleted, substituted, inserted and/or added in the amino acid sequence of SEQ ID NO: 3,
      a CDR2 consisting of an amino acid sequence in which one to two amino acids may be deleted, substituted, inserted and/or added in the amino acid sequence of SEQ ID NO: 4, and
      a CDR3 consisting of an amino acid sequence in which one to two amino acids may be deleted, substituted, inserted and/or added in the amino acid sequence of SEQ ID NO: 5,
         and
      a light chain variable region comprising a CDR1 consisting of an amino acid sequence in which one to two amino acids may be deleted, substituted, inserted and/or added in the amino acid sequence of SEQ ID NO: 7,
      a CDR2 consisting of an amino acid sequence in which one to two amino acids may be deleted, substituted, inserted and/or added in the amino acid sequence of SEQ ID NO: 8, and
      a CDR3 consisting of an amino acid sequence in which one to two amino acids may be deleted, substituted, inserted and/or added in the amino acid sequence of SEQ ID NO: 9, and
      a detection antibody is a monoclonal antibody or antibody fragment thereof against HRG, comprising a heavy chain variable region and a light chain variable region according to any one of the following (i) to (iii):
         (i) a heavy chain variable region comprising a CDR1 consisting of an amino acid sequence in which one to two amino acids may be deleted, substituted, inserted and/or added in the amino acid sequence of SEQ ID NO: 11,
            a CDR2 consisting of an amino acid sequence in which one to two amino acids may be deleted, substituted, inserted and/or added in the amino acid sequence of SEQ ID NO: 12, and
            a CDR3 consisting of an amino acid sequence in which one to two amino acids may be deleted, substituted, inserted and/or added in the amino acid sequence of SEQ ID NO: 13,
               and
            a light chain variable region comprising a CDR1 consisting of an amino acid sequence in which one to two amino acids may be deleted, substituted, inserted and/or added in the amino acid sequence of SEQ ID NO: 15,
            a CDR2 consisting of an amino acid sequence in which one to two amino acids may be deleted, substituted, inserted and/or added in the amino acid sequence of SEQ ID NO: 16, and
            a CDR3 consisting of an amino acid sequence in which one to two amino acids may be deleted, substituted, inserted and/or added in the amino acid sequence of SEQ ID NO: 17, and
         (ii) a heavy chain variable region comprising a CDR1 consisting of an amino acid sequence in which one to two amino acids may be deleted, substituted, inserted and/or added in the amino acid sequence of SEQ ID NO: 11,
            a CDR2 consisting of an amino acid sequence in which one to two amino acids may be deleted, substituted, inserted and/or added in the amino acid sequence of SEQ ID NO: 29, and
            a CDR3 consisting of an amino acid sequence in which one to two amino acids may be deleted, substituted, inserted and/or added in the amino acid sequence of SEQ ID NO: 13,
               and
            a light chain variable region comprising a CDR1 consisting of an amino acid sequence in which one to two amino acids may be deleted, substituted, inserted and/or added in the amino acid sequence of SEQ ID NO: 15,
            a CDR2 consisting of an amino acid sequence in which one to two amino acids may be deleted, substituted, inserted and/or added in the amino acid sequence of SEQ ID NO: 31, and
            a CDR3 consisting of an amino acid sequence in which one to two amino acids may be deleted, substituted, inserted and/or added in the amino acid sequence of SEQ ID NO: 17, and
         (iii) a heavy chain variable region comprising a CDR1 consisting of an amino acid sequence in which one to two amino acids may be deleted, substituted, inserted and/or added in the amino acid sequence of SEQ ID NO: 33,
            a CDR2 consisting of an amino acid sequence in which one to two amino acids may be deleted, substituted, inserted and/or added in the amino acid sequence of SEQ ID NO: 34, and
            a CDR3 consisting of an amino acid sequence in which one to two amino acids may be deleted, substituted, inserted and/or added in the amino acid sequence of SEQ ID NO: 35,
               and
            a light chain variable region comprising a CDR1 consisting of an amino acid sequence in which one to two amino acids may be deleted, substituted, inserted and/or added in the amino acid sequence of SEQ ID NO: 37,
            a CDR2 consisting of an amino acid sequence in which one to two amino acids may be deleted, substituted, inserted and/or added in the amino acid sequence of SEQ ID NO: 38, and
            a CDR3 consisting of an amino acid sequence in which one to two amino acids may be deleted, substituted, inserted and/or added in the amino acid sequence of SEQ ID NO: 39.
(3-1)
   1) The set of monoclonal antibodies or antibody fragments thereof against HRG according to (3), wherein
      a capture antibody is a monoclonal antibody or antibody fragment thereof against HRG, comprising:
      a heavy chain variable region comprising a CDR1 consisting of an amino acid sequence in which one to two amino acids may be deleted, substituted, inserted and/or added in the amino acid sequence of SEQ ID NO: 41,
      a CDR2 consisting of an amino acid sequence in which one to two amino acids may be deleted, substituted, inserted and/or added in the amino acid sequence of SEQ ID NO: 42, and
      a CDR3 consisting of an amino acid sequence in which one to two amino acids may be deleted, substituted, inserted and/or added in the amino acid sequence of SEQ ID NO: 43, and
      a light chain variable region comprising a CDR1 consisting of an amino acid sequence in which one to two amino acids may be deleted, substituted, inserted and/or added in the amino acid sequence of SEQ ID NO: 7,
      a CDR2 consisting of an amino acid sequence in which one to two amino acids may be deleted, substituted, inserted and/or added in the amino acid sequence of SEQ ID NO: 45, and
      a CDR3 consisting of an amino acid sequence in which one to two amino acids may be deleted, substituted, inserted and/or added in the amino acid sequence of SEQ ID NO: 9, and
      a detection antibody is a monoclonal antibody or antibody fragment thereof against HRG, comprising a heavy chain variable region and a light chain variable region according to the following (iv):
         (iv) a heavy chain variable region comprising a CDR1 consisting of an amino acid sequence in which one to two amino acids may be deleted, substituted, inserted and/or added in the amino acid sequence of SEQ ID NO: 33,
      a CDR2 consisting of an amino acid sequence in which one to two amino acids may be deleted, substituted, inserted and/or added in the amino acid sequence of SEQ ID NO: 34, and
      a CDR3 consisting of an amino acid sequence in which one to two amino acids may be deleted, substituted, inserted and/or added in the amino acid sequence of SEQ ID NO: 35,
         and
      a light chain variable region comprising a CDR1 consisting of an amino acid sequence in which one to two amino acids may be deleted, substituted, inserted and/or added in the amino acid sequence of SEQ ID NO: 37,
      a CDR2 consisting of an amino acid sequence in which one to two amino acids may be deleted, substituted, inserted and/or added in the amino acid sequence of SEQ ID NO: 38, and
      a CDR3 consisting of an amino acid sequence in which one to two amino acids may be deleted, substituted, inserted and/or added in the amino acid sequence of SEQ ID NO: 39.
(4) The set of monoclonal antibodies or antibody fragments thereof according to (3), which is selected from the following 1) or 2):
   1) The set of monoclonal antibodies or antibody fragments thereof against HRG according to (3), wherein
      a capture antibody is a monoclonal antibody or antibody fragment thereof against HRG, comprising:
      a heavy chain variable region consisting of an amino acid sequence in which one to two amino acids may be deleted, substituted, inserted and/or added in the amino acid sequence of SEQ ID NO: 40, and
      a light chain variable region consisting of an amino acid sequence in which one to two amino acids may be deleted, substituted, inserted and/or added in the amino acid sequence of SEQ ID NO: 44, and
      a detection antibody is a monoclonal antibody or antibody fragment thereof against HRG, comprising a heavy chain variable region and a light chain variable region according to any one of the following (i) to (v):
         (i) a heavy chain variable region consisting of an amino acid sequence in which one to two amino acids may be deleted, substituted, inserted and/or added in the amino acid sequence of SEQ ID NO: 10, and
            a light chain variable region consisting of an amino acid sequence in which one to two amino acids may be deleted, substituted, inserted and/or added in the amino acid sequence of SEQ ID NO: 14,
         (ii) a heavy chain variable region consisting of an amino acid sequence in which one to two amino acids may be deleted, substituted, inserted and/or added in the amino acid sequence of SEQ ID NO: 18, and
            a light chain variable region consisting of an amino acid sequence in which one to two amino acids may be deleted, substituted, inserted and/or added in the amino acid sequence of SEQ ID NO: 19,
         (iii) a heavy chain variable region consisting of an amino acid sequence in which one to two amino acids may be deleted, substituted, inserted and/or added in the amino acid sequence of SEQ ID NO: 20, and
            a light chain variable region consisting of an amino acid sequence in which one to two amino acids may be deleted, substituted, inserted and/or added in the amino acid sequence of SEQ ID NO: 24,
         (iv) a heavy chain variable region consisting of an amino acid sequence in which one to two amino acids may be deleted, substituted, inserted and/or added in the amino acid sequence of SEQ ID NO: 28, and
            a light chain variable region consisting of an amino acid sequence in which one to two amino acids may be deleted, substituted, inserted and/or added in the amino acid sequence of SEQ ID NO: 30, and
         (v) a heavy chain variable region consisting of an amino acid sequence in which one to two amino acids may be deleted, substituted, inserted and/or added in the amino acid sequence of SEQ ID NO: 32, and
            a light chain variable region consisting of an amino acid sequence in which one to two amino acids may be deleted, substituted, inserted and/or added in the amino acid sequence of SEQ ID NO: 36,
   2) The set of monoclonal antibodies or antibody fragments thereof against HRG according to (3), wherein
      a capture antibody is a monoclonal antibody or antibody fragment thereof against HRG, comprising:
      a heavy chain variable region consisting of an amino acid sequence in which one to two amino acids may be deleted, substituted, inserted and/or added in the amino acid sequence of SEQ ID NO: 2, and
      a light chain variable region consisting of an amino acid sequence in which one to two amino acids may be deleted, substituted, inserted and/or added in the amino acid sequence of SEQ ID NO: 6, and
      a detection antibody is a monoclonal antibody or antibody fragment thereof against HRG, comprising a heavy chain variable region and a light chain variable region according to any one of the following (i) to (iv):
         (i) a heavy chain variable region consisting of an amino acid sequence in which one to two amino acids may be deleted, substituted, inserted and/or added in the amino acid sequence of SEQ ID NO: 10, and
            a light chain variable region consisting of an amino acid sequence in which one to two amino acids may be deleted, substituted, inserted and/or added in the amino acid sequence of SEQ ID NO: 14,
         (ii) a heavy chain variable region consisting of an amino acid sequence in which one to two amino acids may be deleted, substituted, inserted and/or added in the amino acid sequence of SEQ ID NO: 18, and
            a light chain variable region consisting of an amino acid sequence in which one to two amino acids may be deleted, substituted, inserted and/or added in the amino acid sequence of SEQ ID NO: 19,
         (iii) a monoclonal antibody or antibody fragment thereof against HRG, comprising a heavy chain variable region consisting of an amino acid sequence in which one to two amino acids may be deleted, substituted, inserted and/or added in the amino acid sequence of SEQ ID NO: 28, and
            a light chain variable region consisting of an amino acid sequence in which one to two amino acids may be deleted, substituted, inserted and/or added in the amino acid sequence of SEQ ID NO: 30, and
         (iv) a heavy chain variable region consisting of an amino acid sequence in which one to two amino acids may be deleted, substituted, inserted and/or added in the amino acid sequence of SEQ ID NO: 32, and
            a light chain variable region consisting of an amino acid sequence in which one to two amino acids may be deleted, substituted, inserted and/or added in the amino acid sequence of SEQ ID NO: 36.
(4-1)
   1) The set of monoclonal antibodies or antibody fragments thereof according to (4), wherein
      a capture antibody is a monoclonal antibody or antibody fragment thereof against HRG, comprising:
      a heavy chain variable region consisting of an amino acid sequence in which one to two amino acids may be deleted, substituted, inserted and/or added in the amino acid sequence of SEQ ID NO: 40, and
      a light chain variable region consisting of an amino acid sequence in which one to two amino acids may be deleted, substituted, inserted and/or added in the amino acid sequence of SEQ ID NO: 44, and
      a detection antibody is a monoclonal antibody or antibody fragment thereof against HRG, comprising a heavy chain variable region and a light chain variable region according to the following (v):
         (v) a heavy chain variable region consisting of an amino acid sequence in which one to two amino acids may be deleted, substituted, inserted and/or added in the amino acid sequence of SEQ ID NO: 32, and
         a light chain variable region consisting of an amino acid sequence in which one to two amino acids may be deleted, substituted, inserted and/or added in the amino acid sequence of SEQ ID NO: 36.
(5) A kit for detecting or quantifying HRG in a test sample, comprising the monoclonal antibody or antibody fragment thereof according to (1) or (2) or the set of monoclonal antibodies or antibody fragments thereof according to (3), (4), (3-1), or (4-1).
(6) The kit according to (5), wherein the detection or quantification of HRG in a test sample is performed by any immunoassay selected from enzyme immunoassay (ELISA or EIA), fluorescence immunoassay (FIA), radioimmunoassay (RIA), and chemiluminescence immunoassay.
(7) The kit according to (6), wherein the immunoassay is a sandwich ELISA method.
(8) The kit according to (7), wherein the test sample is blood.
(9) The kit according to (8), for detection of sepsis.
(10) A method for measuring HRG in a test sample using a capture antibody and a detection antibody, the method comprising:
   forming a sandwich immune complex containing a capture antibody, HRG, and a detection antibody,
   wherein the capture antibody is a monoclonal antibody or antibody fragment thereof against HRG, comprising:
   a heavy chain variable region comprising a CDR1 consisting of an amino acid sequence in which one to two amino acids may be deleted, substituted, inserted and/or added in the amino acid sequence of SEQ ID NO: 41,
   a CDR2 consisting of an amino acid sequence in which one to two amino acids may be deleted, substituted, inserted and/or added in the amino acid sequence of SEQ ID NO: 42, and
   a CDR3 consisting of an amino acid sequence in which one to two amino acids may be deleted, substituted, inserted and/or added in the amino acid sequence of SEQ ID NO: 43,
      and
   a light chain variable region comprising a CDR1 consisting of an amino acid sequence in which one to two amino acids may be deleted, substituted, inserted and/or added in the amino acid sequence of SEQ ID NO: 7,
   a CDR2 consisting of an amino acid sequence in which one to two amino acids may be deleted, substituted, inserted and/or added in the amino acid sequence of SEQ ID NO: 45, and
   a CDR3 consisting of an amino acid sequence in which one to two amino acids may be deleted, substituted, inserted and/or added in the amino acid sequence of SEQ ID NO: 9, and
   the detection antibody is a monoclonal antibody or antibody fragment thereof against HRG, comprising:
      a heavy chain variable region comprising a CDR1 consisting of an amino acid sequence in which one to two amino acids may be deleted, substituted, inserted and/or added in the amino acid sequence of SEQ ID NO: 33,
      a CDR2 consisting of an amino acid sequence in which one to two amino acids may be deleted, substituted, inserted and/or added in the amino acid sequence of SEQ ID NO: 34, and
      a CDR3 consisting of an amino acid sequence in which one to two amino acids may be deleted, substituted, inserted and/or added in the amino acid sequence of SEQ ID NO: 35,
         and
      a light chain variable region comprising a CDR1 consisting of an amino acid sequence in which one to two amino acids may be deleted, substituted, inserted and/or added in the amino acid sequence of SEQ ID NO: 37,
      a CDR2 consisting of an amino acid sequence in which one to two amino acids may be deleted, substituted, inserted and/or added in the amino acid sequence of SEQ ID NO: 38, and
      a CDR3 consisting of an amino acid sequence in which one to two amino acids may be deleted, substituted, inserted and/or added in the amino acid sequence of SEQ ID NO: 39.
(11) The measurement method according to (10), wherein
   the capture antibody is a monoclonal antibody or antibody fragment thereof against HRG, comprising:
   a heavy chain variable region consisting of an amino acid sequence in which one to two amino acids may be deleted, substituted, inserted and/or added in the amino acid sequence of SEQ ID NO: 40, and
   a light chain variable region consisting of an amino acid sequence in which one to two amino acids may be deleted, substituted, inserted and/or added in the amino acid sequence of SEQ ID NO: 44, and
   the detection antibody is a monoclonal antibody or antibody fragment thereof against HRG, comprising:
      a heavy chain variable region consisting of an amino acid sequence in which one to two amino acids may be deleted, substituted, inserted and/or added in the amino acid sequence of SEQ ID NO: 32, and
      a light chain variable region consisting of an amino acid sequence in which one to two amino acids may be deleted, substituted, inserted and/or added in the amino acid sequence of SEQ ID NO: 36.
(12) The measurement method according to (10) or (11), for detection of sepsis.
(13) A method for measuring HRG in a test sample, comprising using two different antibodies that specifically bind to different epitopes contained in an amino acid sequence at positions 305 to 410 of HRG consisting of the amino acid sequence set forth in SEQ ID NO: 1.
(14) The measurement method according to (13), wherein one of the different epitopes is an epitope containing the amino acid sequence at positions 305 to 325 of HRG consisting of the amino acid sequence set forth in SEQ ID NO: 1.
(14-1) The measurement method according to (13), wherein one of the different epitopes is an epitope containing the amino acid sequence at positions 311 to 319 of HRG consisting of the amino acid sequence set forth in SEQ ID NO: 1.
(15) The measurement method according to (14), wherein one of the different epitopes is an epitope containing the amino acid sequence at positions 391 to 419 of HRG consisting of the amino acid sequence set forth in SEQ ID NO: 1.
(15-1) The measurement method according to (14-1), wherein one of the different epitopes is an epitope containing the amino acid sequence at positions 400 to 410 of HRG consisting of the amino acid sequence set forth in SEQ ID NO: 1.
(16) The measurement method according to (13) to (15), (14-1), or (15-1), wherein
   the two different antibodies is a combination of
   a monoclonal antibody or antibody fragment thereof against HRG, comprising a heavy chain variable region comprising a CDR1 consisting of an amino acid sequence in which one to two amino acids may be deleted, substituted, inserted and/or added in the amino acid sequence of SEQ ID NO: 41,
   a CDR2 consisting of an amino acid sequence in which one to two amino acids may be deleted, substituted, inserted and/or added in the amino acid sequence of SEQ ID NO: 42, and
   a CDR3 consisting of an amino acid sequence of an amino acid sequence in which one to two amino acids may be deleted, substituted, inserted and/or added in the amino acid sequence of SEQ ID NO: 43,
      and
   a light chain variable region comprising a CDR1 consisting of an amino acid sequence in which one to two amino acids may be deleted, substituted, inserted and/or added in the amino acid sequence of SEQ ID NO: 7,
   a CDR2 consisting of an amino acid sequence in which one to two amino acids may be deleted, substituted, inserted and/or added in the amino acid sequence of SEQ ID NO: 45, and
   a CDR3 consisting of an amino acid sequence in which one to two amino acids may be deleted, substituted, inserted and/or added in the amino acid sequence of SEQ ID NO: 9, and
   a monoclonal antibody or antibody fragment thereof against HRG, comprising a heavy chain variable region comprising a CDR1 consisting of an amino acid sequence in which one to two amino acids may be deleted, substituted, inserted and/or added in the amino acid sequence of SEQ ID NO: 33,
   a CDR2 consisting of an amino acid sequence in which one to two amino acids may be deleted, substituted, inserted and/or added in the amino acid sequence of SEQ ID NO: 34, and
   a CDR3 consisting of an amino acid sequence in which one to two amino acids may be deleted, substituted, inserted and/or added in the amino acid sequence of SEQ ID NO: 35,
      and
   a light chain variable region comprising a CDR1 consisting of an amino acid sequence in which one to two amino acids may be deleted, substituted, inserted and/or added in the amino acid sequence of SEQ ID NO: 37,
   a CDR2 consisting of an amino acid sequence in which one to two amino acids may be deleted, substituted, inserted and/or added in the amino acid sequence of SEQ ID NO: 38, and a CDR3 consisting of an amino acid sequence in which one to two amino acids may be deleted, substituted, inserted and/or added in the amino acid sequence of SEQ ID NO: 39.
(17) The measurement method according to (16), wherein
   the two different antibodies is a combination of
   a monoclonal antibody or antibody fragment thereof against HRG, comprising a heavy chain variable region consisting of an amino acid sequence in which one to two amino acids may be deleted, substituted, inserted and/or added in the amino acid sequence of SEQ ID NO: 40, and
   a light chain variable region consisting of an amino acid sequence in which one to two amino acids may be deleted, substituted, inserted and/or added in the amino acid sequence of SEQ ID NO: 44, and
   a monoclonal antibody or antibody fragment thereof against HRG, comprising a heavy chain variable region consisting of an amino acid sequence in which one to two amino acids may be deleted, substituted, inserted and/or added in the amino acid sequence of SEQ ID NO: 32, and
   a light chain variable region consisting of an amino acid sequence in which one to two amino acids may be deleted, substituted, inserted and/or added in the amino acid sequence of SEQ ID NO: 36.
(18) The measurement method according to any one of (13) to (17), (14-1), and (15-1), wherein the method for measuring HRG in a test sample is a sandwich ELISA method.
(19) The measurement method according to (18), wherein the test sample is blood.
(20) The measurement method according to (19), for detection of sepsis.
(21) A method for measuring HRG in a test sample using a set of monoclonal antibodies or antibody fragments thereof against HRG comprising a combination of a capture antibody and a detection antibody, the method comprising:
   (a) reacting a test sample with a capture antibody immobilized on a solid phase in a reaction solution containing an antibody of the same type as the capture antibody or antibody fragment thereof (capture antibody-test sample reaction solution) to produce an immune complex containing the capture antibody immobilized on the solid phase and HRG; and
   (b) measuring the immune complex produced in the (a) using a detection antibody.
(22) The measurement method according to (21), wherein the set is a set of monoclonal antibodies or antibody fragments thereof according to (3), (4), (3-1), or (4-1).
(23) The method according to (21) or (22), for reducing apparent detection sensitivity.
(24) The measurement method according to any one of (21) to (23), further comprising diluting the test sample before the generating an immune complex, in order to reduce a dilution ratio when diluting the test sample.
(25) The kit according to (5), comprising an antibody of the same type as the capture antibody or antibody fragment thereof to be added to the capture antibody-test sample reaction solution.
(26) The kit according to (25), comprising the set of monoclonal antibodies or antibody fragments thereof according to (3), (4), (3-1), or (4-1).
(27) The kit according to (25) or (26), wherein the concentration of the antibody in the reaction solution in the kit is 1.5 to 50 µg/mL.
(28) The kit according to (25) to (27), comprising a document describing the measurement method according to (21).

### [EFFECT OF THE INVENTION]

The monoclonal antibody of the present invention can detect and/or capture HRG in a test sample with good sensitivity and specificity. As a result, a quantitative change in HRG in a test sample can be accurately captured, a change in HRG due to a disease that causes a change in HRG such as sepsis can be detected, and an excellent effect of being applicable to diagnosis of a disease that causes a change in HRG is exhibited.

### [BRIEF DESCRIPTION OF THE DRAWINGS]

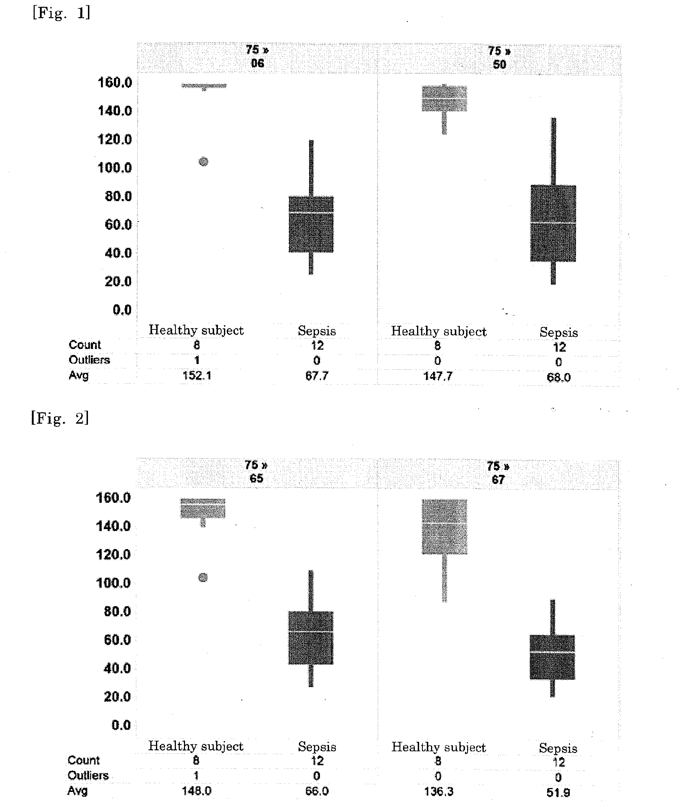

Fig. 1 is a box plot of HRG concentration in plasma for 8 healthy subject specimens and 12 septic patient specimens. The left figure is a combination of capture antibody: 75-2D and detection antibody: 6-12A-10B-9G, and the right figure is a combination of capture antibody: 75-2D and detection antibody: 50-6C-2A. The vertical axis represents the HRG concentration (µg/mL) in plasma, the number of Outliers indicates the number of outliers from the box plot, and Avg indicates the average value of the HRG concentration (µg/mL) of each group (the same applies to Figs. 2 to 5).
Fig. 2 is a box plot of HRG concentration in plasma for 8 healthy subject specimens and 12 septic patient specimens. The left figure is a combination of capture antibody: 75-2D and detection antibody: 65-3A, and the right figure is a combination of capture antibody: 75-2D and detection antibody: 67-8A-5F.

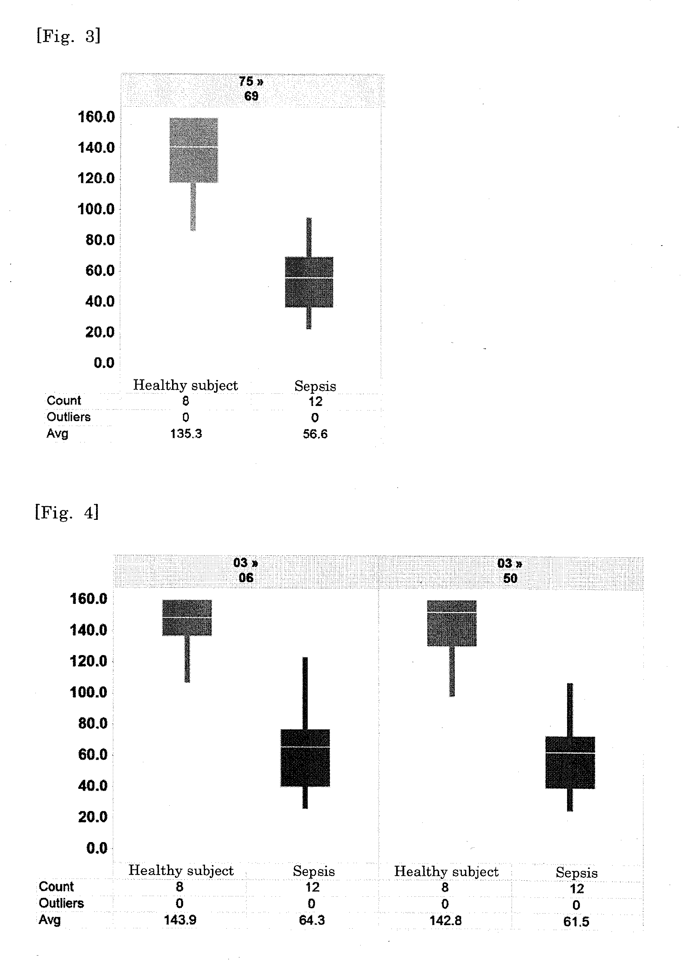

Fig. 3 is a box plot of HRG concentration in plasma for 8 healthy subject specimens and 12 septic patient specimens, showing a combination of capture antibody: 75-2D and detection antibody: 69-1A.
Fig. 4 is a box plot of HRG concentration in plasma for 8 healthy subject specimens and 12 septic patient specimens. The left figure is a combination of capture antibody: 3-4E and detection antibody 6-12A-10B-9G, and the right figure is a combination of capture antibody: 3-4E and detection antibody: 50-6C-2A.

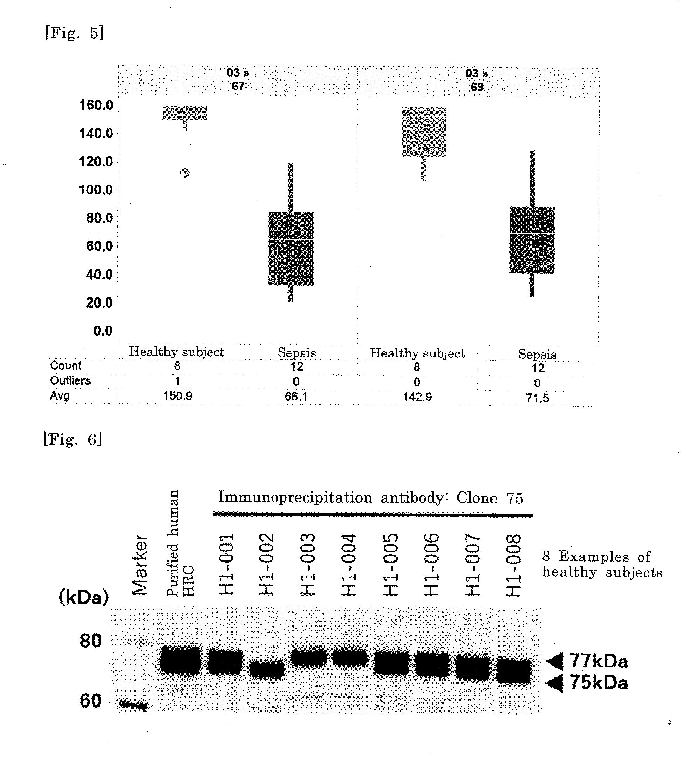

Fig. 5 is a box plot of HRG concentration in plasma for 8 healthy subject specimens and 12 septic patient specimens. The left figure is a combination of capture antibody: 3-4E and detection antibody: 67-8A-5F, and the right figure is a combination of capture antibody: 3-4E and detection antibody: 69-1A.
Fig. 6 shows results of Western blot (WB) analysis of plasma samples of healthy subjects (8 specimens: H1-001 to H1-008) immunoprecipitated with 75-2D with an anti-HRG antibody. The lane of purified human HRG is a result by directly subjecting the purified human HRG described in Example 1 to WB.

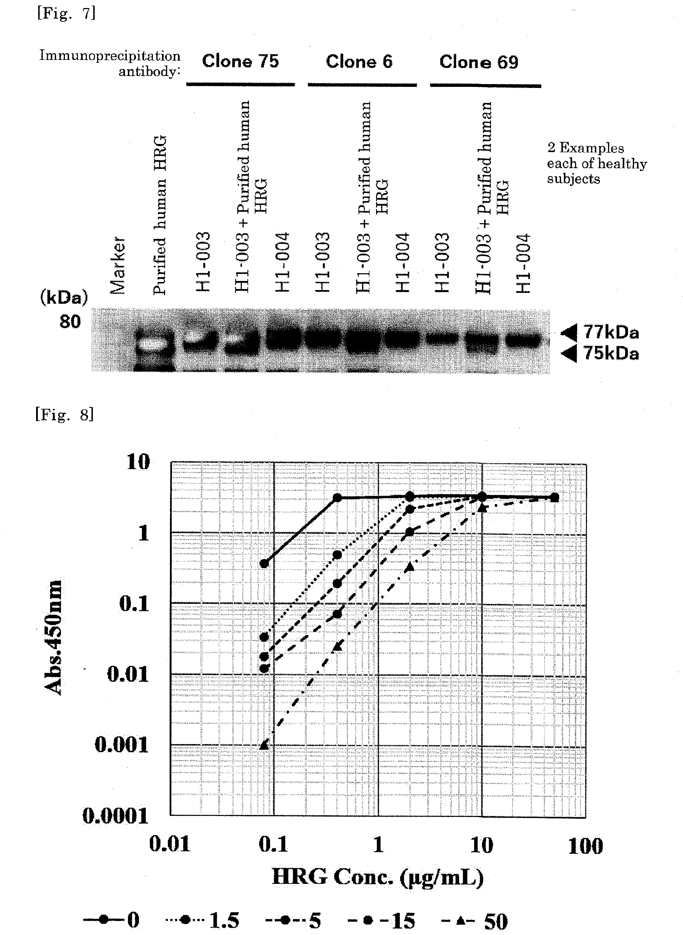

Fig. 7 shows results of WB analysis of plasma of a healthy subject (H1-003), a mixture of plasma of a healthy subject (H1-003) and purified human HRG, and plasma of a healthy subject (H1-004) immunoprecipitated with 75-2D, 6-12A-10B-9G, or 69-1A with an anti-HRG antibody. The lane of purified human HRG is a result by directly subjecting the purified human HRG described in Example 1 to WB.
Fig. 8 shows standard curves of a sandwich ELISA method using a capture antibody (75-2D) and a detection antibody (69-1A) as a combination. Standard curves show those for concentrations of antibody (75-2D) added to the antibody-added buffers of five concentrations of 0, 1.5, 5, 15, 50 pg/mL. The vertical axis represents the absorbance at 450 nm, and the horizontal axis represents the HRG concentration (µg/mL).

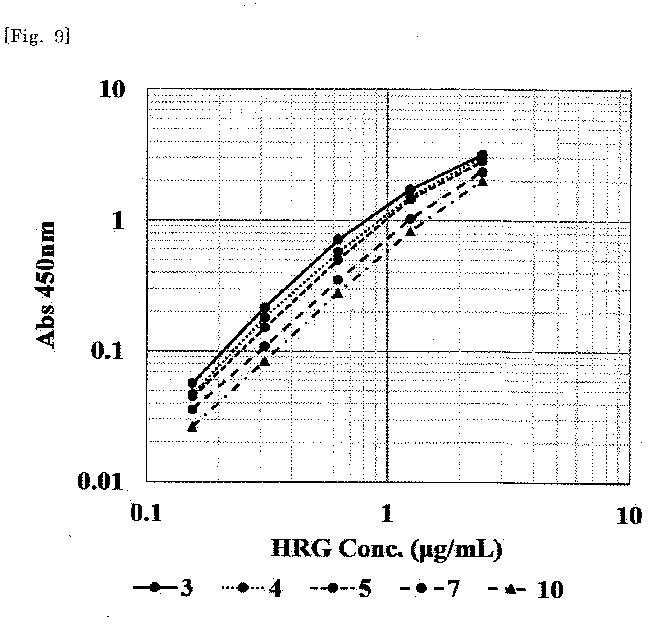

Fig. 9 shows standard curves of a sandwich ELISA method using a capture antibody (75-2D) and a detection antibody (69-1A) as a combination. Standard curves show those for concentrations of antibody (75-2D) added to the antibody-added buffers of five concentrations of 3, 4, 5, 7, 10 µg/mL.

### [MODE FOR CARRYING OUT THE INVENTION]

A human histidine-rich glycoprotein (HRG) that is an antigen of the antibody of the present invention is a peptide consisting of 507 amino acid residues, generated by cleavage of a secretory signal peptide at positions 1 to 18 of a human histidine-rich glycoprotein precursor (NCBI Accession No.: NP_000403.1, SEQ ID NO: 1) consisting of 525 amino acid residues. In the present specification, the amino acid residue number of a human histidine-rich glycoprotein is represented by the residue number of a human histidine-rich glycoprotein precursor that is a precursor thereof.

The monoclonal antibody against HRG of the present invention refers to a monoclonal antibody that specifically binds to HRG, preferably human-derived HRG, and is capable of specifically binding to HRG, and detecting or capturing HRG in a test sample with good sensitivity and specificity. The monoclonal antibody against HRG of the present invention binds to a peptide moiety consisting of 507 amino acids at positions 19 to 525 of HRG consisting of amino acids of SEQ ID NO: 1.

In the present specification, that an antibody "specifically binds to HRG" means that the binding activity of the antibody to HRG is, for example, preferably 2 times or more, 3 times or more, 5 times or more, more preferably 10 times or more, 20 times or more, 30 times or more, and still more preferably 50 times or more, 100 times or more higher than the binding activity to other antigens. The binding activity of the antigen binding molecule to the antigen can be measured and compared by a method known to those skilled in the art, such as ELISA, FACS, and Biacore. For example, it is also possible to measure the binding activity by the method of Example 1 or 2 of the present specification.

The antibody of the present invention can be produced by a known method for producing an antibody, using an antigenic protein containing a full-length or partial fragment of a human histidine-rich glycoprotein (HRG). These antigenic proteins can be prepared by protein expression and purification methods known in the art.

A hybridoma clone producing the monoclonal antibody can be screened for by culturing the hybridoma cells, for example, in a microtiter plate, collecting an antibody in a culture supernatant in a well where growth is seen, measuring the reactivity of the obtained antibody with the antigen of the present invention used in the mouse immune sensitization mentioned above by a measurement method such as RIA, ELISA, or FACS, and selecting a clone producing the monoclonal antibody that exhibits specific binding to the antigen or hapten. In that case, a method can be used which involves immobilizing the antigen on a solid phase, and detecting an antibody in a culture supernatant binding thereto using a secondary antibody labeled with a radioactive material, a fluorescent material, an enzyme, or the like. In the case of using antigen-expressing cells, the hybridoma culture supernatant is added to the cells, and a fluorescently labeled secondary antibody can then be reacted therewith, followed by the measurement of fluorescence intensity of the cells using a fluorescent detection apparatus such as a flow cytometer to detect a monoclonal antibody capable of binding to the antigen of the present invention on the membranes of the cells.

The monoclonal antibody can be produced from the selected hybridoma by culturing the hybridoma in vitro or culturing the hybridoma in the ascitic fluid or the like of a mouse, a rat, a guinea pig, a hamster or a rabbit, etc., preferably a mouse or a rat, more preferably a mouse, and isolating the monoclonal antibody from the obtained culture supernatant or ascetic fluid of the mammal. For the in vitro culture, the hybridoma is grown, maintained and preserved according to various conditions such as the characteristics of the cell type to be cultured, the purpose of a test and research and a culture method and can be cultured using a known nutrient medium used for producing monoclonal antibodies into a culture supernatant, or every nutrient medium prepared from a known basal medium.

Examples of the basal medium include D-MEM medium, RPMI1640 medium, IMDM medium, and ASF104 medium, and the basal medium can contain, for example, serum, hormones, cytokines, and/or various inorganic or organic substances depending on the purpose.

The monoclonal antibody can be isolated and purified, for example, by subjecting the culture supernatant or the ascetic fluid mentioned above to saturated ammonium sulfate, ion-exchange chromatography (DEAE or DE52, etc.), or affinity column chromatography using an anti-immunoglobulin column, a protein A column, or the like.

A recombinant antibody obtained by cloning an antibody gene from antibody-producing cells, for example, hybridomas, integrating the antibody gene into an appropriate vector, and transfecting a host cell with this vector, followed by production by use of a gene recombination technique can be used as the antibody of the present invention (e.g., Carl et al., THERAPEUTIC MONOCLONAL ANTIBODIES, published in 1990).

Specifically, mRNA encoding the variable region (V region) of the antibody is isolated from hybridomas producing the antibody of interest or immunocytes producing the antibody, for example, cells of sensitized lymphocytes immortalized with an oncogene or the like. For the mRNA isolation, total RNA is prepared by a method known in the art, for example, a guanidine ultracentrifugation method (Chirgwin, J.M. et al., Biochemistry (1979) 18, 5294-5299), and the mRNA is prepared using mRNA Purification Kit (manufactured by Pharmacia Inc.) or the like.

cDNA of the antibody V region is synthesized from the obtained mRNA using reverse transcriptase. The synthesis of the cDNA can be performed using AMV Reverse Transcriptase First-strand cDNA Synthesis Kit or the like. 5'-Ampli FINDER RACE Kit (manufactured by Clontech Laboratories, Inc) and PCR-based 5'-RACE (Frohman, M.A. et al., Proc. Natl. Acad. Sci. USA, 1988, Vol. 85, p.8998, etc.) can be used for cDNA synthesis and amplification. A DNA of interest is purified from the obtained PCR product and ligated with vector DNA to prepare a recombinant vector. The nucleotide sequence of the DNA of interest is confirmed by a method known in the art, for example, a deoxy method.

Provided that the DNA encoding the variable region (V region) of the antibody of interest is successfully obtained, this DNA is linked to DNA encoding the desired antibody constant region (C region) and the resultant is integrated into an expression vector. Alternatively, the DNA encoding the V region of the antibody may be integrated into an expression vector containing the DNA of the antibody C region. To produce an antibody used in the present invention, the antibody gene is incorporated into an expression vector such that it is expressed under the control of an expression control region, e.g., an enhancer/promoter. The expression vector can then be used to transform a host cell and express the antibody.

The sequence of the constant region of the monoclonal antibody of the present invention is not particularly limited as long as binding to HRG is not lost.

For the expression of the antibody gene, DNA encoding the heavy chain (H chain) and DNA encoding the light chain (L chain) of the antibody may be separately integrated into expression vectors, with which a host is co-transformed, or the DNA encoding the H chain and the DNA encoding the L chain may be integrated into a single expression vector, with which a host is transformed (see WO94/11523).

A so-called phage display technique (Nature Biotechnology 23, 1105 (2005)) can also be used as a method, other than those described above, for preparing the antibody of the present invention. Specifically, for example, an antibody gene library prepared by a method known in the art using human or animal (e.g., rabbit, mouse, rat, or hamster) B lymphocytes as a material, or an antibody gene library completely synthesized by selection and engineering from a human or animal germ line sequence is displayed on, for example, bacteriophages, E. coil, yeast or animal cell surface, or liposomes. In this respect, examples of the form of the antibody to be displayed on the cell surface include IgG molecules, IgM molecules, Fab fragments, and single-strand Fv (scFv) fragments.

The antibody fragment gene thus obtained can be recombined with a corresponding region of an IgG antibody gene by a method known in the art to obtain an antibody gene. Then, the gene thus obtained can be integrated into an appropriate vector, with which a host is transfected, followed by the production of the antibody by use of a gene recombination technique (e.g., Carl et al., THERAPEUTIC MONOCLONAL ANTIBODIES, published in 1990).

The type of antibody fragment of the monoclonal antibody of the present invention is not particularly limited, and examples thereof include Fab (fragment of antigen binding), Fab', F(ab')₂, Fv, and ScFv. An overview of these antibody fragments is given in Hudson, P.J. et al., Nat. Med. 9 (2003) 129-134 and Plueckthun, A., In: The Pharmacology of Monoclonal Antibodies, Vol. 113, Rosenburg and Moore (eds.), Springer-Verlag, New York (1994), 269-315.

When the antibody or antibody fragment thereof of the present invention is used as a detection antibody, a label such as a microparticle, a fluorescent substance, an enzyme, biotin, a radiolabeled form, or a magnetic particle is preferably added from the viewpoint of use for detecting HRG.

The microparticle is not particularly limited as long as the antigen-binding property of the antibody or antibody fragment thereof is not impaired, and examples thereof include metal colloidal particles such as colloidal gold, and colored particles such as colored latex particles.

The fluorescent substance is not particularly limited as long as the antigen-binding property of the antibody or antibody fragment thereof is not impaired, and examples thereof include rhodamine-based, coumarin-based, oxazine-based, carbopyronine-based, cyanine-based, pyrromethene-based, naphthalene-based, biphenyl-based, anthracene-based, phenanthrene-based, pyrene-based, carbazole-based, Cy-based, EvoBlue-based, fluorescein-based, and derivatives thereof.

The enzyme is not particularly limited as long as the antigen-binding property of the antibody or antibody fragment thereof is not impaired, and examples thereof include horseradish peroxidase (HRP), alkaline phosphatase, peroxidase, β-D-galactosidase, and microperoxidase.

The radiolabeled form is not particularly limited, and examples thereof include ³H, ¹⁴C, ¹⁵N, ³⁵S, ⁹⁰Y, ⁹⁹Tc, ¹¹¹In, ¹²⁵I, and ¹³¹I.

A tag can be added to the antibody or antibody fragment thereof of the present invention. Examples of such a tag include FLAG, HA (hemagglutinin), GST (glutathione-S-transferase), Myc, and polyhistidine (6×His tag, etc.). These tags can be used for immobilization, purification, detection, and the like of an antibody. The tagged antibody or antibody fragment can be produced, for example, by preparing a polynucleotide encoding the antibody or antibody fragment thereof of the present invention to which a polynucleotide encoding a tag was added and introducing it into an in vivo protein expression system such as an animal cell or an in vitro protein expression system such as a cell-free translation system.

The present invention includes a set of monoclonal antibodies or antibody fragments thereof against HRG, for use in measurement of HRG in a test sample. The set of antibodies includes a combination of two types of antibodies that bind to an epitope of HRG consisting of the amino acid sequence set forth in SEQ ID NO: 1, and one antibody functions as a capture antibody and the other antibody functions as a detection antibody, whereby the HRG in a test sample can be measured with good sensitivity and specificity. The capture antibody refers to an antibody that specifically binds to HRG in a test sample, and is immobilized on a solid phase to capture HRG in a test substance on the solid phase. The detection antibody refers to an antibody that specifically binds to HRG in a test sample and has a labeling substance, and provides a signal detectable via the labeling substance. The capture antibody and the detection antibody may be antibody fragments.

In the present specification, the measurement includes both a qualitative detection method (detection) and a quantitative detection method (quantification).

Preferred examples of a set of monoclonal antibodies or antibody fragments thereof against HRG for use in measurement of HRG in a test sample in the present invention include the following combinations.

A set of antibodies, in which the capture antibody is 75-2D, and
the detection antibody is 6-12A-10B-9G, 50-6C-2A, 65-3A, 67-8A-5F, or 69-1A, or
a set of antibodies, in which the capture antibody is 3-4E, and
the detection antibody is 6-12A-10B-9G, 50-6C-2A, 67-8A-5F, or 69-1A.

A particularly preferred set is the following combination.

A set of antibodies, in which the capture antibody is 75-2D, and the detection antibody is 69-1A.

A kit for detecting or quantifying HRG in a test sample of the present invention includes the set of monoclonal antibodies or antibody fragments thereof of the present invention, and is used for detecting or quantifying HRG in a test sample. The test sample is not particularly limited, and examples thereof include blood, urine, saliva, lymphatic fluid, semen, tear fluid, intraocular fluid, vitreous humor, feces, sweat, or other body fluid, or a separated matter or an extract thereof (plasma, etc.), but blood or a separated matter or an extract thereof is preferable, and plasma is more preferable.

Examples of a diluent for diluting the test sample include a buffer solution (e.g., phosphate buffer, imidazole buffer, triethanolamine-hydrochloric acid, citrate buffer, and Good's buffer), and may include a protein stabilizer (e.g., bovine serum albumin (BSA)), a protease inhibitor(e.g., aprotinin), sodium chloride, and a preservative (e.g., sodium azide, ProClin, and antibiotics).

The kit of the present invention is not particularly limited, and can be appropriately selected according to the detection or measurement method to be used, but it is preferable to further include one or more kinds of measuring instruments and/or reagents for pretreatment, detection, or measurement of the measurement target, in addition to an anti-HRG antibody and antibody fragment thereof.

The kit of the present invention may include, as other reagents, one or more selected from water, HRG as a standard substance, a pH buffer component, a surfactant, a salt (sodium chloride, potassium chloride, etc.), a saccharide (sucrose, trehalose, sugar alcohol, etc.), a blocking agent (bovine serum albumin (BSA), casein, serum, etc.), a substrate for color development or fluorescence detection, and the like, in addition to the monoclonal antibody or antibody fragment thereof against HRG. These components are preferably provided in the form of a liquid (aqueous solution).

The pH buffer component is not particularly limited, and examples thereof include an acid such as phosphoric acid, boric acid, acetic acid, citric acid, formic acid, or cacodylic acid, or a salt thereof; an amino acid such as glycine; and a Good's buffer such as trishydroxyaminomethane (Tris), HEPES, or MES.

The surfactant is not particularly limited, but a nonionic surfactant is preferable, and any of an ester ether type, an ester type, and an ether type can be used. More specific examples thereof include Tween 20, Tween 40, Tween 80, Triton-X100, polyoxyethylene (60) sorbitan monostearate, polyoxyethylene (65) sorbitan tristearate, polyoxyethylene (80) sorbitan monooleate, polyoxyethylene alkylphenyl ether, Nonidet P-40, and CHAPS. These surfactants may be used alone or in combination of two or more thereof.

The detection or quantification of HRG in the kit of the present invention is preferably measured by any immunoassay selected from enzyme immunoassay (ELISA or EIA), fluorescence immunoassay (FIA), radioimmunoassay (RIA), and chemiluminescence immunoassay. In particular, measurement by sandwich ELISA is preferable.

When the detection or measurement method is an immunochromatography method or an ELISA method, the measuring instrument preferably contains a solid phase substrate carrying the capture antibody.

When the detection or measurement method is an immunochromatography method or an ELISA method, the labeled antibody may be contained in the measuring instrument, or may be contained, for example, in a sample tube separately from the measuring instrument.

In an embodiment of the immunochromatography kit, the measuring instrument is not particularly limited, but preferably includes a water-absorbent solid phase substrate on which one or more types of anti-HRG antibodies or antibody fragments thereof are immobilized. Examples of the material of the solid phase substrate include nitrocellulose.

The sandwich ELISA method is performed, for example, as follows. First, one antibody (capture antibody) is immobilized on a well surface of an ELISA plate. Then, after blocking is performed to prevent non-specific adsorption to the well surface, a sample is added, and HRG in the sample is brought into contact with the antibody to form a complex. After proteins that have not bound to the antibody are removed by washing, the other labeled antibody (detection antibody) is added to the well and brought into contact with HRG to form a complex, and detection and quantification are performed by the labeling. Quantification can be performed by a known method such as quantifying a signal detected based on the used label using a calibration curve (in the present specification, it may be described as a standard curve.) prepared using a standard substance of HRG.

In the sandwich ELISA method, a labeled antibody can be used, and for example, an enzyme-labeled antibody can be used. Examples of the enzyme used for labeling include peroxidase, alkaline phosphatase, glucose oxidase, and β-galactosidase. As a substrate agent used for detecting the enzyme, an appropriate one is selected according to the selected labeling enzyme. For example, when peroxidase is selected as the enzyme, o-phenylenediamine (OPD), tetramethylbenzidine (TMB), or the like is used, and when alkaline phosphatase is selected, p-nitrophenyl phosphate (PNPP) or the like is used. Also, as a reaction stop solution and a substrate solution, conventionally known ones can be appropriately used without particular limitation depending on the selected enzyme.

As another embodiment, the present invention includes the following method for measuring HRG in a test sample (alternative method).

### [Alternative method]

A method for measuring HRG in a test sample using a set of monoclonal antibodies or antibody fragments thereof against HRG comprising a combination of a capture antibody and a detection antibody, the method comprising:
(a) reacting a test sample with a capture antibody immobilized on a solid phase in a reaction solution containing an antibody of the same type as the capture antibody or antibody fragment thereof to produce an immune complex containing the capture antibody immobilized on the solid phase and HRG; and
(b) measuring the immune complex produced in the (a) using a detection antibody.

A method for measuring HRG in a test sample in which the (a) of the alternative method is replaced with another step "reacting a test sample with a capture antibody immobilized on a solid phase in a reaction solution not containing an antibody of the same type as the capture antibody or antibody fragment thereof to produce an immune complex containing the capture antibody immobilized on the solid phase and HRG" is referred to as a normal method in the present specification. In addition, in a method for measuring HRG in a test sample using a set of monoclonal antibodies or antibody fragments thereof against HRG comprising a combination of a capture antibody and a detection antibody, including the above-described alternative method and normal method, a reaction solution when (the HRG of) the test sample is reacted with the capture antibody immobilized on the solid phase is referred to as a capture antibody-test sample reaction solution in the present specification. The "antibody-added buffer" in Examples of the present specification is used as a capture antibody-test sample reaction solution.

In the above alternative method, a standard substance of HRG to be measured for preparing a calibration curve is also subjected to the measurement method comprising the steps (a) and (b) as in the test sample, and measured values are calculated to prepare a calibration curve.

In the above alternative method, it is considered that the antibody of the same type as the capture antibody or antibody fragment thereof in the capture antibody-test sample reaction solution competitively inhibits the HRG to be measured in the test sample and the capture antibody immobilized on the solid phase from producing an immune complex. As a result, the production amount of the immune complex of the HRG and the capture antibody immobilized on the solid phase can be reduced, and the apparent detection sensitivity can be lowered. In immunoassay such as ELISA, there is a concentration range of a measurement target suitable for quantitative measurement (HRG in the present invention), and when the measurement target is present at a high concentration in a test sample such as human plasma like HRG, it is necessary to dilute the sample to be measured at a very high magnification (for example, 5,000 times) in order to adjust the sample to the concentration range. By using this alternative method, the dilution ratio of such a test sample can be reduced, and as a result, the measurement error can be reduced by reducing the number of times of dilution.

In the above alternative method, the "antibody of the same type as the capture antibody or antibody fragment thereof' in the capture antibody-test sample reaction solution means an antibody having the same properties as the antibody used for the capture antibody or antibody fragment thereof. As the antibody of the same type as the capture antibody or antibody fragment thereof, an antibody having the same epitope as the capture antibody or antibody fragment thereof is preferable, an antibody having the same amino acid sequences of a heavy chain variable region containing CDR1 to CDR3 and a light chain variable region containing CDR1 to CDR3 as the capture antibody or antibody fragment thereof is more preferable, and an antibody having the same amino acid sequences of a heavy chain variable region and a light chain variable region as the capture antibody or antibody fragment thereof is still more preferable.

In the above alternative method, a preferable set of monoclonal antibodies or antibody fragments thereof against HRG for use in measurement of HRG in a test sample is the following combination.

A set of antibodies, in which the capture antibody is 75-2D, and
the detection antibody is 6-12A-10B-9G, 50-6C-2A, 65-3A, 67-8A-5F, or 69-1A, or
a set of antibodies, in which the capture antibody is 3-4E, and
the detection antibody is 6-12A-10B-9G, 50-6C-2A, 67-8A-5F, or 69-1A.

A particularly preferred set is the following combination.

A set of antibodies, in which the capture antibody is 75-2D, and the detection antibody is 69-1A.

In the preferred set of monoclonal antibodies or antibody fragments thereof against HRG in the alternative method, the "antibody of the same type as the capture antibody or antibody fragment thereof' in the capture antibody-test sample reaction solution is preferably the same antibody as the capture antibody used in each set of antibodies. For example, in the set of antibodies in which the capture antibody is 75-2D and the detection antibody is 69-1A, the antibody to be added to the capture antibody-test sample reaction solution is preferably 75-2D.

Considering that the effective absorbance range in general ELISA is about 0.01 to 3.00, in the above alternative method, the preferred concentration of the antibody of the same type as the capture antibody or antibody fragment thereof in the capture antibody-test sample reaction solution is 1.5 to 50 pg/mL, more preferred concentration is 1.5 to 15 pg/mL, and most preferred is 7 pg/mL. The antibody concentration (µg/mL) mentioned here is a concentration assuming an antibody having a normal heavy chain, light chain, and constant region, and is corrected to a concentration (µg/mL) corresponding to the molecular weight of the antibody when the molecular weight of the antibody such decreases as an antibody fragment.

The alternative method may be used for the kit for detecting or quantifying HRG in a test sample of the present invention, and when the kit is for the alternative method, it is preferable that a measurement method by the alternative method is described in a document or the like attached to the kit. Furthermore, the document may include a description of conditions for performing an alternative method such as "the capture antibody is added so that the concentration of the capture antibody in the capture antibody-test sample reaction solution is 1.5 to 50 pg/mL".

### [EXAMPLES]

Hereinafter, the present invention will be explained in more detail by way of Examples of the present invention, but the present invention is not limited by them.

### Example 1: Preparation of anti-HRG mouse monoclonal antibodies

### (1) Preparation of antigens

HRG was purified by Ni-NTA (nickel-nitrilotriacetic acid) affinity chromatography and high-performance liquid chromatography (anion exchange column (monodisperse hydrophilic polymer beads: Mono Q)) using human plasma (240 mL) as a starting material. The purified sample was dialyzed against phosphate buffered saline (Phosphate Buffered Saline: 1× PBS(-), hereinafter referred to as "PBS".), and afforded preparations containing 500 to 1000 pg/ml (5 mL) of HRG were stored. The purified sample according to the present method was used as "human purified HRG" also in Examples 6 to 13.

### (2) Preparation of hybridoma and selection of reactive antibody.

BALB/c mice were immunized with the prepared antigens, and hybridomas producing antibodies against human HRG were produced by the method described in Sado Y et al., Acta Histochem. Cytochem. 39: 89-94 (2006). From the obtained hybridomas, strains producing a reactive antibody with an antigen were selected by an ELISA method. The selected hybridomas were cloned by limiting dilution method, and strains stably producing antibodies against human HRG were further selected.

### (3) Purification of monoclonal antibodies

After expansion to a certain concentration in conventional growth medium, the hybridomas were gradually diluted with a serum-free medium and finally acclimated to the serum-free medium. The acclimated hybridomas were gradually expanded and finally cultured in 300 mL. The culture supernatant was collected when the number of living cells come to 20% or less while confirming the growth state of the hybridomas.

For the obtained culture supernatant was run a Protein A column according to a conventional method, followed by washing sufficiently with a binding buffer, and then the antibody was eluted with an elution buffer. The eluate containing the antibody was neutralized followed by dialyzing twice against 5 L of D-PBS. The dialyzed antibody solution was aseptically filtered using a 0.2 pm membrane filter in a clean bench.

### Example 2: Evaluating combinations of antibodies applicable to sandwich ELISA system

In 21 types of the purified anti-HRG monoclonal antibodies, combinations of antibodies by which a sandwich ELISA system could work were evaluated by the following method.

### (1) Test conditions

### (1.1) Preparation of detection antibody

The purified anti-HRG antibody was biotin-labeled using Biotin Labeling Kit-SH (Cat# LK09, DOJINDO LABORATORIES) to obtain a detector antibody.

### (1.2) Preparation of standard substance for evaluation (human purified HRG protein)

496 pL of 0.1% BSA-containing PBS was mixed with 4.0 pL of human purified HRG protein (1 mg/mL) to afford a human purified HRG protein solution (10 pg/mL). Furthermore, 4,950 pL of 0.1% BSA-containing PBS was mixed with 50 pL of human purified HRG protein (10 µg/mL) followed by 100-fold dilution to afford a human purified HRG protein solution (100 ng/mL).

### (1.3) Preparation of capture antibody

As for a capture antibody, the purified anti-HRG antibody was diluted with a 0.2 M carbonate buffer to give a capture antibody solution (10 µg/mL).

### (2) Test method

The capture antibody solution was dispensed at 25 pL per well into 384-well Maxisorp plates (NUNC), and the plates were incubated at room temperature for 3 hours. The plates were washed twice with 120 pL of washing solution per well (0.05%Tween 20-containing PBS). A blocking solution (1% BSA-containing PBS) was dispensed at 30 pL per well, and the plates were incubated at room temperature for 3 hours. The blocking solution was removed followed by dispensing the standard substance for evaluation at 20 pL per well (n=2), and the plates were incubated at room temperature for 3 hours. After washing the plates four times with the washing solution as described above, the solution of each detector antibody was dispensed at 10 pL per well, followed by dispensing a horseradish peroxidase-streptavidin solution (0.05 pg/mL) at 10 pL per well, and the plates were incubated at room temperature for 1 hour. After washing the plates four times with the washing solution as described above, a chromogenic substrate solution (TMB (3,3',5,5'-tetramethylbenzidine)) was dispensed at 20 pL per well followed by incubating the plates at room temperature for 10 minutes, then a reaction stop solution 0.5 M H₂SO₄ was added at 20 pL/well, and the absorbance at 450 nm was measured.

### (3) Results

The results regarding the evaluation of combinations chosen from 21 types of anti-HRG monoclonal antibodies are shown in Table 1. In Table 1, the absorbance at a wavelength of 450 nm of each combination was determined as follows: less than 1.0 OD: -, 1.0 OD or more and less than 2.0 OD: +, 2.0 OD or more and less than 3.0 OD: ++, and 3.0 OD or more : +++. The combinations of antibodies which could construct a sandwich ELISA system having enough level of absorbance change were 86 sets evaluated as ++ or +++ in Table 1.

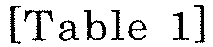

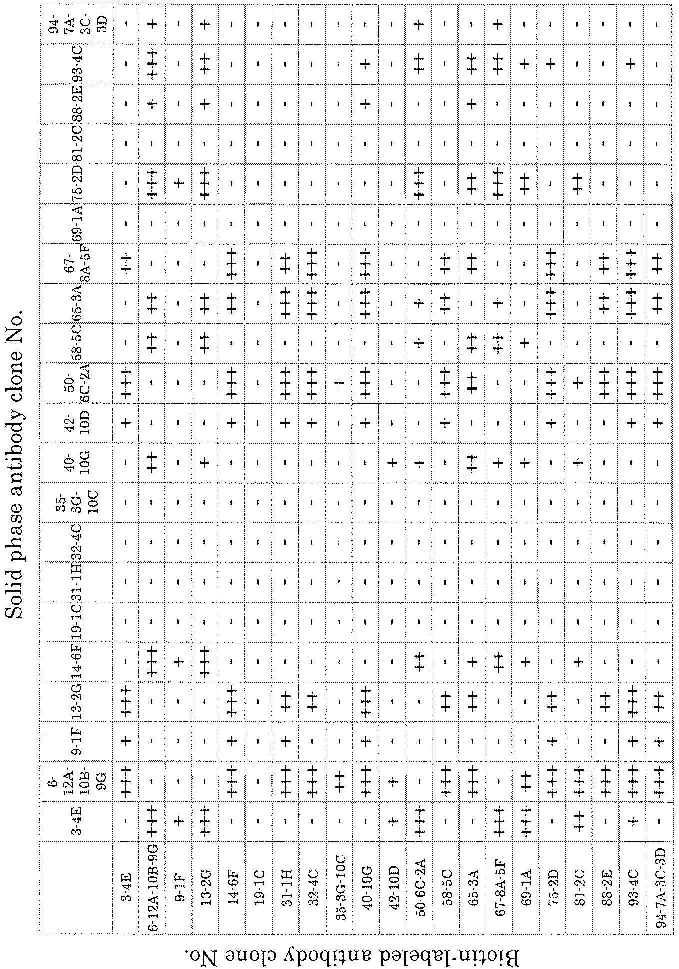

### Example 3: Reactivity evaluation of each antibody in sandwich ELISA system

The 86 combinations of antibodies applicable to a sandwich ELISA system was evaluated for reactivity with HRG.

### Test conditions

### (1.1) Preparation of detector antibody (same as above)

The purified anti-HRG antibody was biotin-labeled using Biotin Labeling Kit-SH (Cat# LK09, DOJINDO LABORATORIES) to give a detection antibody.

### (1.2) Preparation of standard substance for calibration curve (human purified HRG protein)

The human HRG solution was afforded by mixing 396 µL of 0.1% BSA-containing PBS with 4.0 pL of human purified HRG (1 mg/mL). The human HRG solution was diluted 100-fold with 0.1% BSA-containing PBS to set the upper limit of a calibration curve as100 ng/mL.

The human HRG solution was further diluted to prepare a dilution series (50 ng/mL, 25 ng/mL, 12.5 ng/mL, 6.25 ng/mL, 3.125 ng/mL, 1.56 ng/mL, and 0 ng/mL).

### (1.3) Preparation of capture antibody (same as above)

The purified anti-HRG antibody was diluted with 0.2 M carbonate buffer to obtain a capture antibody solution (10 pg/mL).

### (2) Test method

The capture antibody solution was dispensed at 25 pL per well into 384-well Maxisorp plates (NUNC) followed by incubating at room temperature for 3 hours. The plates were washed twice with 120 pL of washing solution per well (0.05%Tween 20-containing PBS). A blocking solution (1% BSA-containing PBS) was dispensed at 30 pL per well followed by incubating at room temperature for 3 hours. After removal of the blocking solution, the standard substance for calibration curve diluted in 7 steps was dispensed at 20 pL per well (n=2) followed by incubating at 4°C overnight. After washing the plates four times with the washing solution as described above, the solution of each detection antibody was dispensed at 10 pL per well, and a horseradish peroxidase-streptavidin solution (0.05 pg/mL) was dispensed at 10 µL per well followed by incubating at room temperature for 2 hours. After washing the plates four times with the washing solution as described above, a chromogenic substrate solution (TMB (3,3',5,5'-tetramethylbenzidine)) was dispensed at 20 µL per well followed by incubating at room temperature for 10 minutes, then a reaction stop solution 0.5 M H₂SO₄ was added at 20 pL/well for the measurement of absorbance at 450 nm. The measured absorbance value with respect to the HRG concentration of the standard substance were plotted to give calibration curves.

### (3) Results

It has been found that any combination of anti-HRG antibodies is useful as the antibody-material for ELISA method detecting HRG.

### Example 4: Measurement of patient specimens

Using each antibody prepared in Example 1, blood HRG values of 12 septic patient specimens and 8 healthy adult subjects were measured by the sandwich ELISA system described in Example 3, and correlation analysis of measured values was performed by a sandwich ELISA system constructed with a combination of different antibodies.

### (1) Test conditions

### (1.1) Biological sample

Blood was collected from 12 septic patients and 8 healthy adult subjects to give plasma specimens (20 specimens). Each plasma specimen was diluted 1,600-fold with 0.1% BSA-containing PBS to be used for measurement.

### (1.2) Detector antibody, capture antibody and standard substance for calibration curve

A biotin-labeled anti-HRG antibody prepared in the same manner as described in Example 3 was used as a detection antibody. Each detector antibody was diluted with an antibody dilution solution (0.1% BSA-containing PBS) to afford a solution of each detection antibody (2.5 pg/mL for each). The standard substance for calibration curve and the capture antibody were prepared in the same manner as described in Example 3.

### (2) Test method

The capture antibody solution was dispensed at 25 pL per well into 384-well Maxisorp plates (NUNC) and the plates were incubated at room temperature for 3 hours. The plates were washed and blocked by a similar manner to Example 3. After removal of the blocking solution, the standard substance for calibration curve diluted in 7 steps and the plasma specimen diluted 1,600 fold were dispensed at 20 pL per well (n=2) followed by incubating at 4°C overnight. In the same manner as Example 3, the solution of each detector antibody was dispensed at 10 µL per well after washing the plates four times with the washing solution, and a horseradish peroxidase-streptavidin solution (0.05 pg/mL) was dispensed at 10 pL per well followed by incubating at room temperature for 2 hours. After washing the plates four times with the washing solution as described above, a chromogenic substrate solution (TMB (3,3',5,5'-tetramethylbenzidine)) was dispensed at 20 pL per well followed by incubating at room temperature for 10 minutes, then a reaction stop solution 0.5 M H₂SO₄ was added at 20 µL/well for the measurement of absorbance at 450 nm.

### (3) Data processing

A calibration curve was prepared for each combination of antibodies. From the calibration curve, the value of HRG was obtained for each 1,600-fold diluted plasma specimen. The HRG concentration value in each plasma specimen was defined as the result of the measured HRG value multiplied by 1600. When the value obtained from the calibration curve was negative, it was regarded as "0 pg/mL". In addition, since the maximum value of the measurement concentration range was 160 pg/mL, a sample measured to have a concentration of 160 pg/mL or more was regarded as "160 µg/mL".

### (4) Results

When the difference in plasma HRG concentration between the specimens of septic patients and healthy adult subjects was measured using 75-2D as a capture antibody and 6-12A-10B-9G, 50-6C-2A, 65-3A, 67-8A-5F, or 69-1A as a detector antibody, or when measured using 3-4E as a capture antibody and 6-12A-10B-9G, 50-6C-2A, 67-8A-5F, or 69-1A as a detector antibody, a large difference was found in plasma HRG concentration between the septic patients and healthy adult subjects (Figs. 1 to 5). The significant difference in HRG concentration in plasma specimens of septic patients and healthy adult subjects was p = 0.00002 or less by Welch's t test in any combination of antibodies.

### Example 4: Analysis of amino acid sequence of anti-HRG antibody

Base sequence analysis of the variable region of each anti-HRG antibody was performed for 7 clones shown in Table 1 which anti-HRG antibodies were prepared in Example 1. Total RNA was purified from a frozen stock of hybridoma producing anti-HRG antibody (1 × 10⁶ cells/vial, 1 vial) followed by the synthesis of cDNA. An antibody variable region was amplified by PCR using a degenerate primer by a template of the synthesized cDNA. The amplified product afforded by PCR was subjected to sequence analysis to examine the variable regions.

The amino acid sequences of the anti-HRG antibodies given by the above analysis are shown in Table 2.

**[Table 2]**

| mAb clone No. | | SEQ ID No. | | SEQ ID No. | | SEQ ID No. | | SEQ ID No. |
|---|---|---|---|---|---|---|---|---|
| 3-4E | Heavy chain | 2 | CDR1 | 3 | Light chain | 6 | CDR1 | 7 |
| | | | CDR2 | 4 | | | CDR2 | 8 |
| | | | CDR3 | 5 | | | CDR3 | 9 |
| 6-12A-10B-9G | Heavy chain | 10 | CDR1 | 11 | Light chain | 14 | CDR1 | 15 |
| | | | CDR2 | 12 | | | CDR2 | 16 |
| | | | CDR3 | 13 | | | CDR3 | 17 |
| 50-6C-2A | Heavy chain | 18 | CDR1 | 11 | Light chain | 19 | CDR1 | 15 |
| | | | CDR2 | 12 | | | CDR2 | 16 |
| | | | CDR3 | 13 | | | CDR3 | 17 |
| 65-3A | Heavy chain | 20 | CDR1 | 21 | Light chain | 24 | CDR1 | 25 |
| | | | CDR2 | 22 | | | CDR2 | 26 |
| | | | CDR3 | 23 | | | CDR3 | 27 |
| 67-8A-5F | Heavy chain | 28 | CDR1 | 11 | Light chain | 30 | CDR1 | 15 |
| | | | CDR2 | 29 | | | CDR2 | 31 |
| | | | CDR3 | 13 | | | CDR3 | 17 |
| 69-1A | Heavy chain | 32 | CDR1 | 33 | Light chain | 36 | CDR1 | 37 |
| | | | CDR2 | 34 | | | CDR2 | 38 |
| | | | CDR3 | 35 | | | CDR3 | 39 |
| 75-2D | Heavy chain | 40 | CDR1 | 41 | Light chain | 44 | CDR1 | 7 |
| | | | CDR2 | 42 | | | CDR2 | 45 |
| | | | CDR3 | 43 | | | CDR3 | 9 |

The amino acid sequences of anti-HRG monoclonal antibody 69-1A are as follows.
Heavy chain variable region: 
EVKLEESGGGLVQPGGSMKLSCAASGFTFSDAWMDWVRQSPEKGLEWVAEIRSKA
NNHGTYYAESVKGRFTISRDDSKSSVYLRMNSLSAEDTGIYYCTRPFAYWGQGTLVT
VSA (SEQ ID NO: 32)
Heavy chain CDR1: GFTFSDAWMD (SEQ ID NO: 33)
Heavy chain CDR2: EIRSKANNHGTYYAESV (SEQ ID NO: 34)
Heavy chain CDR3: PFAY (SEQ ID NO: 35)
Light chain variable region: 
DVVVTQTPLSLPVSLGDQASISCRSSQSLVHIDGNTYFHWYVQKPGQSPKLLIYKVSN
RFSGVPDRFSGSGSGTDFTLKISRVEAEDLGVYFCSQSTHVPLTFGAGTKLELK (SEQ
ID NO: 36)
Light chain CDR1: RSSQSLVHIDGNTYFH (SEQ ID NO: 37)
Light chain CDR2: KVSNRFS (SEQ ID NO: 38)
Light chain CDR3: SQSTHVPLT (SEQ ID NO: 39)

The amino acid sequences of anti-HRG monoclonal antibody 75-2D are as follows.
Heavy chain variable region: 
QVQLQQSGAELMKPGASVKISCKATGYTFSSYWIEWVKQRPGHGLEWIGEILPGSGR
TNYNEKFKGKATFTADTSSNTAYMQLSSLTSEDSAVYYCARYSYFYYFDYWGQGTTL
TVSS (SEQ ID NO: 40)
Heavy chain CDR1: GYTFSSY (SEQ ID NO: 41)
Heavy chain CDR2: EILPGSGRTNYNE (SEQ ID NO: 42)
Heavy chain CDR3: YSYFYYFDY (SEQ ID NO: 43)
Light chain variable region: 
DNVLTQSPAIMSASLGERVTMTCTASSSVSSSYLHWYQQKPGSSPKLWIYSTSNLASG
VPARFSGSGSGTSYSLTISSMEAEDAATYYCHQYHRSPWTFGGGTKLEIK (SEQ ID
NO: 44)
 DNVLTQSPAIMSASLGERVTMTCTASSSVSSSYLHWYQQKPGSSPKLWIYSTSNLASG VPARFSGSGSGTSYSLTISSMEAEDAATYYCHQYHRSPWTFGGGTKLEIK (SEQ ID NO: 44
Light chain CDR1: TASSSVSSSYLH (SEQ ID NO: 7)
Light chain CDR2: STSNLAS (SEQ ID NO: 45)
Light chain CDR3: HQYHRSPWT (SEQ ID NO: 9)

### Example 5: Determination of binding sites recognized by anti-HRG monoclonal antibodies

It was confirmed an epitope to which amino acid sequence the anti-HRG monoclonal antibodies 69-1A and 75-2D bind in a human HRG protein having the amino acid sequence shown in SEQ ID NO: 1.

### (1) Preparation of peptide microarrays

An overlapping peptide scanning method was performed to find out specific antibody-binding peptide epitopes. The amino acid sequence of the HRG protein was obtained in NCBI protein database. PEPperPrint (registered trademark) (Heidelberg, Germany) was commissioned to prepare PEPperCHIP peptide microarrays designed from HRG protein sequences. Each peptide consisted of an overlapping peptide library having 15 amino acid residues overlapping 14 amino acid residues and was extended at the N- and C-terminus with a neutral GSGSGSG linker to ensure that the peptides were separated from each other. On each microarray, 1,050 peptides derived from HRG protein were duplicately printed (2,100 spots total), and HA peptide and c-Myc peptide as control peptides were placed on a frame of each array. After treatment of 21 types of anti-HRG monoclonal antibodies, it stained with an anti-mouse IgG polyclonal secondary antibody followed by reading fluorescence intensity on a scanner to quantify protein-antibody binding.

### (2) Test method

Each microarray was processed as follows. Before incubation with a sample, a standard buffer was applied on slides followed by treating with a blocking buffer. The microarray was treated with a fluorescently-labeled anti-mouse IgG secondary antibody followed by the measurement of background fluorescence intensity caused by non-specific interactions using a scanner.
The microarray was incubated with the 21 types of anti-HRG monoclonal antibodies. The microarray was stained with a fluorescently labeled anti-mouse IgG secondary antibody. The fluorescence intensity at each position was measured by a scanner.

### (3) Analysis method

The analysis was performed by making use of analysis software, PEPSlide analyzer. This software brings up the relation of a fluorescence intensity value and a background fluorescence value shown at each spot to the peptide sequence corresponding to each spot. It gave the corrected fluorescence intensity, average, median, and standard deviation of the median by the calculation.

### (4) Results

HRG monoclonal antibody clone No. 69-1A showed good binding capability to peptides #2 to #8 in Table 3. This No. 69-1A did not bind strongly to the other peptides. As such it was considered that the binding site of the antibody was included in the domain of positions 305 to 325 of the amino acid sequence set forth in SEQ ID NO: 1. In particular, it was suggested that the antibody binds to a human HRG protein at a consensus amino acid sequence included in peptides #2 to #8 (PLPQGPPPL: SEQ ID NO: 56) as an epitope. This epitope corresponds to 311st to 319th amino acid sequence from a N-terminus of the amino acid sequence shown in SEQ ID NO: 1.

**[Table 3]**

| No. | Sequence | Position of each peptide in SEQ ID No: 1 | SEQ ID No. of each peptide |
|---|---|---|---|
| Peptide #1 | RDHSHGPPLPQGPPP | 304 to 318 | 47 |
| Peptide #2 | DHSHGPPLPQGPPPL | 305 to 319 | 48 |
| Peptide #3 | HSHGPPLPQGPPPLL | 306 to 320 | 49 |
| Peptide #4 | SHGPPLPQGPPPLLP | 307 to 321 | 50 |
| Peptide #5 | HGPPLPQGPPPLLPM | 308 to 322 | 51 |
| Peptide #6 | GPPLPQGPPPLLPMS | 309 to 323 | 52 |
| Peptide #7 | PPLPQGPPPLLPMSC | 310 to 324 | 53 |
| Peptide #8 | PLPQGPPPLLPMSCS | 311 to 325 | 54 |
| Peptide #9 | LPQGPPPLLPMSCSS | 312 to 326 | 55 |

The anti-HRG monoclonal antibody clone No. 75-2D showed binding capability to peptides #11 to #26 in Table 4, and it was particularly strong with peptides #17 to #21.
From this, it was considered that the binding site of the antibody was included in the domain of positions 391 to 419 of the amino acid sequence presented in SEQ ID NO: 1. In particular, it was suggested that the antibody binds to a human HRG protein at a consensus amino acid sequence contained in peptides #17 to #21 (HPHGHHPHCHD: SEQ ID NO: 73) as an epitope. This epitope corresponds to 400th to 410th amino acid sequence from a N-terminus of the amino acid sequence shown in SEQ ID NO: 1.

**[Table 4]**

| No. | Sequence | Position of each peptide in SEQ ID No: 1 | SEQ ID No. of each peptide |
|---|---|---|---|
| Peptide #11 | HPHGHHPHGHHPHGH | 391 to 404 | 57 |
| Peptide #12 | PHGHHPHGHHPHGHH | 392 to 405 | 58 |
| Peptide #13 | HGHHPHGHHPHGHHP | 393 to 406 | 59 |
| Peptide #14 | GHHPHGHHPHGHHPH | 394 to 407 | 60 |
| Peptide #15 | HHPHGHHPHGHHPHC | 395 to 408 | 61 |
| Peptide #16 | HPHGHHPHGHHPHCH | 396 to 409 | 62 |
| Peptide #17 | PHGHHPHGHHPHCHD | 396 to 410 | 63 |
| Peptide #18 | HGHHPHGHHPHCHDF | 397 to 411 | 64 |
| Peptide #19 | GHHPHGHHPHCHDFQ | 398 to 412 | 65 |
| Peptide #20 | HHPHGHHPHCHDFQD | 399 to 413 | 66 |
| Peptide #21 | HPHGHHPHCHDFQDY | 400 to 414 | 67 |
| Peptide #22 | PHGHHPHCHDFQDYG | 401 to 415 | 68 |
| Peptide #23 | HGHHPHCHDFQDYGP | 402 to 416 | 69 |
| Peptide #24 | GHHPHCHDFQDYGPC | 403 to 417 | 70 |
| Peptide #25 | HHPHCHDFQDYGPCD | 404 to 418 | 71 |
| Peptide #26 | HPHCHDFQDYGPCDP | 405 to 419 | 72 |

### Example 6: Capture by immunoprecipitation with anti-HRG monoclonal antibody

### (1) Immunoprecipitation

As a method for using an anti-HRG antibody, it was examined about the capability for purification of HRG by immunoprecipitation. Also, a commercially available antibody having better ability of immunoprecipitation than any other available one was preliminarily singled out for comparison of that capability. Specifically, an antibody from R&D Systems (Clone No. 227901, Cat No. MAB1869) was used. The purified human plasma HRG and healthy subjects plasma shown in Example 1 were used for the specimen of immunoprecipitation.

### (2) Test method

The purified anti-HRG antibodies (three antibodies: 75-2D, 6-12A-10B-9G and 69-1A) were biotin-labeled using Biotin Labeling Kit-SH (Cat# LK09, DOJINDO LABORATORIES) to afford capture antibodies. The prepared capture antibodies were dissolved in PBS followed by the reaction with Dynabeads M-280 Streptavidin at room temperature for 2 hours. Subsequently, it was washed with 0.1% BSA-containing PBS using a magnet to afford antibody-immobilized beads.
(2-1) To the antibody-immobilized beads of 75-2D was added 25 µL of healthy subjects plasma (8 specimens: H1-001 to H1-008) by diluting three times with PBS to react at room temperature for 4 hours.
(2-2) As another experiment, to the immobilized beads of 3 antibodies of 75-2D, 6-12A-10B-9G, and 69-1A, 25 pL of plasma of healthy subjects (H1-003, 004) and a mixture of 25 pL of plasma of a healthy subject (H1-003) and purified human HRG was diluted three times with PBS and added, and the mixture was reacted at room temperature for 4 hours.

After the reaction completion, the beads were afforded by washing with 0.1% BSA-containing PBS using a magnet. HRG captured on the beads was extracted followed by evaluation by Western blotting (WB). WB was performed using a commercially available anti-HRG antibody from R&D Systems (Clone No. 227901, Cat No. MAB1869), ECL Anti-mouse IgG and Horseradish Peroxidase linked whole antibody. Fig. 6 shows the result of WB in the above (2-1), and Fig. 7 shows the result of WB in the above (2-2).

### (3) Results

As depicted in Fig. 6, it was found that 75-2D could recognize isoforms of two HRGs having different molecular weights by about 2 kDa which were presented in human plasma and could immunoprecipitate. As depicted in Fig. 7, it was found that 6-12A-10B-9G and 69-1A could recognize two HRG isoforms having different molecular weights by about 2 kDa which were presented in human plasma, 77kDa and 75kDa, and could immunoprecipitate, similarly to 75-2D.

### Example 7: Comparison of reactivity by different concentration of capture antibody added to the reaction mixture of capture antibody-test sample in sandwich ELISA system (Examination of alternative method 1)

A sandwich ELISA system was constructed by the following method using two types of purified anti-HRG antibodies (combination of the capture antibody (75-2D) and the detector antibody (69-1A)). Subsequently, in the case that the capture antibody was contained in a reaction mixture of capture antibody and test sample, the difference of reactivity depending on the concentration of the added capture antibody was examined.

### (1) Test conditions

### (1-1) Preparation of buffer

50mM PB containing 0.3 M NaCl, 0.1% BSA, 0.01% mouse γ-globulin, 0.05% Tween 20 and 0.1% Procline (phosphate buffer, pH 7.4, hereinafter referred to as "PB")

### (1-2) Preparation of antibody diluent

PBS containing 0.4% BlockAce, 0.1% Procline

### (1-3) Preparation of washing solution

PBS containing 0.05% Tween 20, 0.1% Procline

### (1-4) Preparation of capture antibody

The purified anti-HRG antibody (75-2D) was diluted with PBS to afford a capture antibody solution (5 pg/mL).

### (1-5) Preparation of detection antibody

The purified anti-HRG antibody (69-1A) was horseradish peroxidase (HRP) labeled and diluted with an antibody diluent to afford a detection antibody solution (75 ng/mL).

### (1-6) Preparation of standard substance for drawing a calibration curve (human purified HRG protein)

Human purified HRG (1,000 pg/mL) was diluted with the buffer to afford a standard substance solution for drawing a calibration curve (50 pg/mL). The upper limit of the calibration curve was set to 50 pg/mL, and the standard substance solution for drawing a calibration curve (50 pg/mL) was diluted five-fold with the buffer to afford a standard substance solution for drawing a calibration curve (10 pg/mL). The standard substance solution for calibration curve was further diluted to prepare a 5-fold dilution series (2, 0.4, and 0.08 pg/mL).

### (1-7) Antibody-added buffer

The purified anti-HRG antibody (75-2D) was added to the buffer of (1-1) above so as to have each concentration of 0, 1.5, 5, 15, and 50 pg/mL to prepare antibody-added buffer.

### (2) Test method

The capture antibody solution of (1.4) above was dispensed into 96-well Maxisorp plates (ThermoFisher, NUNC) at 150 pL per well followed by incubating at room temperature for 2 hours. The plates were washed twice with 300 pL of washing solution per well. A blocking solution (1% BSA-containing PBS) was dispensed at 300 pL per well. The plates were incubated at 4°C overnight or more. The prepared plates were stored at 4°C. After removal of the blocking solution, the plates were washed twice with 300 pL of washing solution per well and then the standard substance for drawing a calibration curve diluted in 5 steps was dispensed at 25 pL per well (n=2).

The antibody-added buffer (1.5 pg/mL) was dispensed at 100 pL per well to each well to which the standard substance for calibration curve was added. Similarly, the antibody-added buffers of four concentrations of 0, 5, 15, and 50 pg/mL were each also dispensed at 100 pL per well to each well to which the standard substance for calibration curve was added.

The plates were incubated at room temperature for 2 hours. After washing the plates three times with 300 pL of washing solution per well, the solution of detector antibody was dispensed at 100 pL per well followed by incubating at room temperature for 1 hour. After washing the plates three times with the washing solution as described above, a chromogenic substrate solution (TMB (3,3',5,5'-tetramethylbenzidine)) was dispensed at 100 pL per well followed by incubating at room temperature for 15 minutes, then a reaction stop solution 0.5 M H₂SO₄ was added at 50 µL/well for the measurement of absorbance at 450 nm. The measured absorbance value with respect to the HRG concentration of the standard substance were plotted to give calibration curves.

### (3) Results

The calibration curves drawn for the conditions under which each of the antibody-added buffers having five concentrations was added are shown in Fig. 8. Considering that the effective absorbance range in a common ELISA is approximately 0.01 to 3.00, the antibody-added buffer concentration range is supposed to be from 1.5 pg/mL to 50 pg/mL at which the absorbance level for the sandwich ELISA system could be achievable. From the economical point of view, a smaller amount of antibody usage is preferrable, and this viewpoint suggests that the concentration of the capture antibody in the antibody-added buffer is preferably in the range from 1.5 pg/mL to 15 pg/mL.

### Example 8: Optimization trial for antibody concentration of antibody-added buffer added to reaction solution in sandwich ELISA system (Examination of alternative method 2)

By changing concentration of the antibody-added buffer added to the reaction solution in the range of 3 µg/mL to 10 pg/mL, the optimal antibody concentration of the antibody-added buffer was examined.

### Test conditions

For the preparation of the buffer solution, the antibody diluent, the washing solution, the capture and detector antibody, the test was performed using the same practices as those described in Example 7.

### (1-2) Preparation of standard substance for drawing calibration curve (human purified HRG protein)

Purified HRG (1,000 pg/mL) was diluted with the buffer to afford a standard substance solution for drawing calibration curve (2.5 pg/mL). The upper limit of the calibration curve was set to 2.5 pg/mL, and the standard substance solution for calibration curve (2.5 pg/mL) was diluted twice with the buffer to afford a standard substance solution for drawing calibration curve (1.25 pg/mL). The standard substance solution was further undergoing dilution to afford a 2-fold dilution series (0.63, 0.32, and 0.16 pg/mL) for drawing calibration curve.

### (1-3) Antibody-added buffer

The purified anti-HRG antibody (75-2D) was added to the buffer so as to have concentrations of 3, 4, 5, 7, and 10 µg/mL.

### (2) Test method

The capture antibody solution was dispensed at 150 pL per well into 96-well Maxisorp plates (ThermoFisher, NUNC) followed by incubating at room temperature for 2 hours. The plates were washed twice with 300 pL of washing solution per well. A blocking solution (1% BSA-containing PBS) was dispensed at 300 µL per well. The plates were incubated at 4°C overnight or more. The prepared plates were stored at 4°C. After removal of the blocking solution, the plates were washed twice with 300 µL of washing solution per well followed by dispensing the standard substance diluted in 5 steps at 25 pL per well (n=2) for calibration curve. The standard substance for calibration curve was added to each well followed by dispensing the antibody-added buffer (3 pg/mL) at 100 pL per well. Similarly, the standard substance for calibration curve was added to each well followed by dispensing the antibody-added buffers of four concentrations of 4, 5, 7, and 10 pg/mL at 100 pL per well. The plates were incubated at room temperature for 2 hours. After washing the plates three times with 300 pL of washing solution per well, the detection antibody solution was dispensed at 100 pL per well followed by incubating at room temperature for 1 hour. After washing the plates three times with the washing solution as described above, a chromogenic substrate solution (TMB (3,3',5,5'-tetramethylbenzidine)) was dispensed at 100 pL per well followed by incubating at room temperature for 15 minutes, then a reaction stop solution 0.5 M H₂SO₄ was added at 50 pL/well for measuring the absorbance at 450 nm. The measured absorbance value with respect to the HRG concentration of the standard substance were plotted to give calibration curves.

### (3) Results

The calibration curves each generated for the conditions under which each of the antibody-added buffers having five concentrations was added are shown in Fig. 9. The absorbance range at a level in which the sandwich ELISA system was constructed was achieved at any antibody addition concentration. It was found that when the specimen dilution ratio was set to 101-fold, the optimal antibody-added buffer concentration was 7 pg/mL as the antibody-added buffer concentration capable of more quantitatively measuring plasma HRG having concentration range from 0.16 to 2.5 pg/mL.

### Example 9: Examination of quantitative range when 7 µg/mL antibody-added buffer was added to reaction solution in sandwich ELISA system (Examination of alternative method 3) To set the quantitative range of the sandwich ELISA system, 0.04 to 2.5 µg/mL human purified HRG was measured.

### (1) Test conditions

For the preparation of the buffer solution, the antibody diluent, the washing solution, the capture and detector antibody, the test was performed using the same practices as those described in Example 7.(1-1) Antibody-added buffer

The purified anti-HRG antibody (75-2D) was added to the buffer to adjust concentration as 7 pg/mL.

### (1-2) Preparation of human purified HRG dilution examples

Human purified HRG (1,000 pg/mL) was diluted with the buffer to afford a purified HRG solution (2.5 pg/mL). The purified HRG solution (2.5 pg/mL) was diluted twice with the buffer to afford a purified HRG solution (1.25 µg/mL). The purified HRG solution was further diluted to prepare a 2-fold dilution series (0.63, 0.32, 0.16, 0.08, and 0.04 pg/mL). The buffer without human purified HRG was used for a blank solution (HRG concentration 0.00 pg/mL).(2) Test method

The capture antibody solution was dispensed at 150 µL per well into 96-well Maxisorp plates (ThermoFisher, NUNC) followed by incubating at room temperature for 2 hours. The plates were washed twice with 300 pL of washing solution per well. A blocking solution (1% BSA-containing PBS) was dispensed at 300 µL per well. The plates were incubated at 4°C overnight or more. The prepared plates were stored at 4°C.

After removal of the blocking solution, the plates were washed twice with 300 pL of washing solution per well followed by dispensing purified HRG dilution series (7-step dilutions from 2.5 pg/mL to 0.04 pg/mL) solution or the blank solution at 25 µL per well (n=5).

The human purified HRG dilution series (7 steps) solution or the blank solution was added to each well followed by dispensing the antibody-added buffer (7 pg/mL) at 100 pL per well.

The plates were incubated at room temperature for 2 hours. After washing the plates three times with 300 pL of washing solution per well, the detector antibody solution was dispensed at 100 pL per well followed by incubating at room temperature for 1 hour. After washing the plates three times with the washing solution as described above, a chromogenic substrate solution (TMB (3,3',5,5'-tetramethylbenzidine)) was dispensed at 100 pL per well followed by incubating at room temperature for 15 minutes, then a reaction stop solution 0.5 M H₂SO₄ was added at 50 pL/well for measuring the absorbance at 450 nm (Table 5).(3) Results

To set the quantitative range, measurement of 0.04 to 2.5 pg/mL purified HRG was performed, it was performed simultaneous measurement 5 times giving result that the coefficient of variation of within-run reproducibility was 2.2% to 7.3%. The quantitative range was a concentration range in which the average ± 3SD of the absorbance of the blank and the average ± 3SD of the absorbance of the corresponding concentration did not overlap. The satisfying concentration range for the above was 0.16 to 2.5 µg/mL (Table 5).

**[Table 5]**

| HRG Concentration (µg/mL) | 0.00 | 0.04 | 0.08 | 0.16 | 0.31 | 0.63 | 1.25 | 2.50 |
|---|---|---|---|---|---|---|---|---|
| Absorbance 450nm | 0.059 | 0.067 | 0.069 | 0.096 | 0.169 | 0.434 | 1.201 | 2.459 |
| | 0.058 | 0.065 | 0.069 | 0.087 | 0.171 | 0.423 | 1.079 | 2.582 |
| | 0.053 | 0.063 | 0.068 | 0.083 | 0.179 | 0.417 | 1.038 | 2.510 |
| | 0.054 | 0.062 | 0.066 | 0.091 | 0.155 | 0.369 | 1.199 | 2.513 |
| | 0.053 | 0.055 | 0.066 | 0.088 | 0.159 | 0.436 | 1.055 | 2.609 |
| Average value | 0.055 | 0.062 | 0.068 | 0.089 | 0.167 | 0.416 | 1.114 | 2.535 |
| SD | 0.003 | 0.005 | 0.002 | 0.005 | 0.010 | 0.027 | 0.079 | 0.060 |
| CV | 5.2% | 7.3% | 2.2% | 5.4% | 5.8% | 6.6% | 7.1% | 2.4% |
| (1) 0 Average + 3SD | 0.064 | | | | | | | |
| (2) Average - 3SD | | 0.049 | 0.063 | 0.074 | 0.138 | 0.334 | 0.876 | 2.354 |
| (2) - (1) | | -0.015 | -0.001 | **0.010** | **0.074** | **0.270** | **0.812** | **2.290** |

From this, it gave the preferable quantitative range of the sandwich ELISA system set which was determined to be 0.16 to 2.5 pg/mL.

### Example 10: Examination of reproducibility of sandwich ELISA system (Examination of alternative method 4)

Since the quantitativeness was confirmed at 0.16 to 2.5 pg/mL, the reproducibility of the measurement system was examined under the calibration curve set to the range of 0.16 to 2.5 µg/mL. The within-run reproducibility was given by a coefficient of variation within the same measurement time when quintuple measurement was performed, and the between-run reproducibility was given by a coefficient of variation between measurements when the same specimen was measured four times in different days afforded by double measurement. The inter-measurer difference reproducibility was given by a coefficient of variation between measurements when the same specimen was measured three times in duplicate by different measurements..

### (1) Test conditions

For the preparation of the buffer solution, the antibody diluent, the washing solution, the capture and detector antibody, the test was performed using the same practices as those described in Example 7.

### (1-1) Preparation of standard substance for drawing calibration curve (human purified HRG protein)

Human purified HRG (1,000 µg/mL) was diluted with the buffer to afford a standard substance solution for drawing calibration curve (2.5 pg/mL). Since the upper limit of the calibration curve was set to 2.5 pg/mL, the standard substance solution for drawing calibration curve (2.5 pg/mL) was diluted twice with the buffer to afford a standard substance solution for drawing calibration curve (1.25 pg/mL). The standard substance solution for drawing calibration curve was further diluted to prepare a 2-fold dilution series (0.63, 0.32, and 0.16 pg/mL).

### (1-2) Antibody-added buffer

As in Example 9, the purified anti-HRG antibody (75-2D) was added to the buffer adjusting a concentration of 7 µg/mL.

### (1-3) Biological sample

Pooled plasma specimens (two specimens) of healthy adult subjects were diluted 101-fold with the buffer to be used for measurement.

### (2) Test method

The capture antibody solution was dispensed at 150 pL per well into 96-well Maxisorp plates (ThermoFisher, NUNC) followed by incubating at room temperature for 2 hours. The plates were washed twice with 300 pL of washing solution per well. A blocking solution (1% BSA-containing PBS) was dispensed at 300 µL per well. The plates were incubated at 4°C overnight or more. The prepared plates were stored at 4°C.

After removal of the blocking solution, the plates were washed twice with 300 pL of washing solution per well followed by dispensing the standard substance solution for calibration curve diluted in 5 steps at 25 pL per well (n=2). For the within-run reproducibility, biological samples (two specimens) were dispensed at 25 pL per well (n=5). For the between-run reproducibility, biological samples (two specimens) were dispensed at 25 pL per well (n=2) on each day. For the inter-measurer reproducibility, biological samples (two specimens) were dispensed at 25 µL per well (n=2) by each measurer.

The standard substance solution for drawing calibration curve and the biological samples were added to each well followed by dispensing the antibody-added buffer (7 pg/mL) at 100 pL per well.

The plates were incubated at room temperature for 2 hours. After washing the plates three times with 300 pL of washing solution per well, the solution of detection antibody was dispensed at 100 pL per well followed by incubating at room temperature for 1 hour. After washing the plates three times with the washing solution as described above, a chromogenic substrate solution (TMB (3,3',5,5'-tetramethylbenzidine)) was dispensed at 100 µL per well followed by incubating at room temperature for 15 minutes, then a reaction stop solution 0.5 M H₂SO₄ was added at 50 pL/well for measuring the absorbance at 450 nm. The measured absorbance value with respect to the HRG concentration of the standard substance were plotted to prepare calibration curves.

### (3) Results

As a result of examining the reproducibility of the measurement system using the biological samples (two specimens), the coefficient of variation of within-run reproducibility was 3.6% for specimen No. 1 and 1.8% for specimen No. 2 (Table 6), and the coefficient of variation of between-run reproducibility was 7.3% for specimen No. 1 and 9.3% for specimen No. 2 (Table 7), which were favorable values. The coefficient of variation of inter-measurer reproducibility was also 8.4% for specimen No. 1 and 6.6% for specimen No. 2 (Table 8). It was found that the sandwich ELISA system using the 7 µg/mL antibody-added buffer to the reaction solution is an HRG quantification system having high reproducibility.

**[Table 6]**

| HRG Concentration (µg/mL) | Number of measurements | | | | | Average value | SD | CV (%) |
|---|---|---|---|---|---|---|---|---|
| | 1 | 2 | 3 | 4 | 5 | | | |
| Specimen No. 1 | 73.1 | 76.0 | 76.4 | 79.0 | 80.1 | 76.9 | 2.7 | 3.6 |
| Specimen No. 2 | 101.1 | 104.5 | 102.9 | 111.2 | 104,5 | 102.9 | 1.9 | 1.8 |

**[Table 7]**

| HRG Concentration (µg/mL) | Number of measurements | | | | Average value | SD | CV (%) |
|---|---|---|---|---|---|---|---|
| | 1 | 2 | 3 | 4 | | | |
| Specimen No. 1 | 85.3 | 85.9 | 73.1 | 79.9 | 81.1 | 6.0 | 7.3 |
| Specimen No. 2 | 124.6 | 122.9 | 101.1 | 113.5 | 115.5 | 10.8 | 9.3 |

**[Table 8]**

| HRG Concentration (µg/mL) | Measurer | | | Average value | SD | CV (%) |
|---|---|---|---|---|---|---|
| | No.1 | No.2 | No.3 | | | |
| Specimen No. 1 | 85.3 | 74.8 | 87.9 | 82.7 | 7.0 | 8.4 |
| Specimen No. 2 | 124.6 | 116.0 | 132.4 | 124.3 | 8.2 | 6.6 |

### Example 11: Examination of effect of interfering substance (Examination of alternative method 5)

The effect of the interfering substance on the sandwich ELISA system was evaluated, and it was examined whether plasma specimens containing the interfering substance can be used for HRG quantification.(1) Test conditions

For the preparation of the buffer solution, the antibody diluent, the washing solution, the capture and detector antibody, the test was performed using the same practices as those described in Example 7. For the preparation of the standard substance for drawing calibration curve (human purified HRG protein) and the antibody-added buffer, the test was performed using the same practices as shown in Example 10.(1-1) Interfering substance

Interference Check-A Plus [free bilirubin, conjugated bilirubin, hemolyzed hemoglobin, chyle] (Sysmex Corporation) was prepared according to the standard procedure on the package insert. Pooled plasma specimens of healthy adult subjects were used for base specimens.

Specifically, free bilirubin and free bilirubin (blank) dissolved in a reagent bottle were added in an amount of 1/10 with respect to the base specimen. These were used as sample A and sample B. A dilution series were prepared according to the procedure in which the sample A and the sample B were prepared so that mixing ratios of the sample A and the sample B were 0 : 10, 2 : 8, 4 : 6, 6 : 4, 8 : 2, and 10 : 0, and these were used for solutions containing an interfering substance. Similarly, solutions of conjugated bilirubin, hemolyzed hemoglobin, and chyle were also prepared.

### (1-2) Preparation of solutions containing interfering substance

The solutions containing an interfering substance were diluted 101-fold with the buffer to be used for measurement.(2) Test method

The capture antibody solution was dispensed at 150 pL per well into 96-well Maxisorp plates (ThermoFisher, NUNC) followed by incubating at room temperature for 2 hours. The plates were washed twice with 300 pL of washing solution per well. A blocking solution (1% BSA-containing PBS) was dispensed at 300 pL per well. The plates were incubated at 4°C overnight or more. The prepared plates were stored at 4°C. After removal of the blocking solution, the plates were washed twice with 300 pL of washing solution per well followed by dispensing the standard substance solution for drawing calibration curve diluted in 5 steps and the diluted solution containing an interfering substance at 25 pL per well (n=2). The standard substance solution for calibration curve and the diluted solution containing an interfering substance were added to each well followed by dispensing the antibody-added buffer (7 pg/mL) at 100 pL per well

The plates were incubated at room temperature for 2 hours. After washing the plates three times with 300 pL of washing solution per well, the solution of detection antibody was dispensed at 100 pL per well followed by incubating at room temperature for 1 hour. After washing the plates three times with the washing solution as described above, a chromogenic substrate solution (TMB (3,3',5,5'-tetramethylbenzidine)) was dispensed at 100 pL per well followed by incubating at room temperature for 15 minutes, then a reaction stop solution 0.5 M H₂SO₄ was added at 50 µL/well for measuring the absorbance at 450 nm. The measured absorbance value with respect to the HRG concentration of the standard substance were plotted to give calibration curves.(3) Results

When the free bilirubin was at 5 concentrations of 4.1, 8.2, 12.3, 16.4, and 20.5 mg/dL, the HRG concentration of the specimens gave 92 to 101% values compared with the case of 0 mg/dL free bilirubin. (Table 9)

**[Table 9]**

| HRG Concentration (µg/mL) | Free bilirubin (mg/dL) | | | | | |
|---|---|---|---|---|---|---|
| | 0 | 4.1 | 8.2 | 12.3 | 16.4 | 20.5 |
| Specimen No. 2 | 121 | 122 | 112 | 116 | 114 | 111 |
| To 0 (mg/dL) | - | 101% | 92% | 96% | 94% | 92% |

In the case that the conjugated bilirubin was at 5 concentrations of 3.9, 7.8, 11.8, 15.7, and 19.6 mg/dL, the HRG concentration of the specimen was 105 to 116% with respect to the HRG concentration for the case that conjugated bilirubin was 0 mg/dL. (Table 10)

**[Table 10]**

| HRG Concentration (µg/mL) | Conjugated bilirubin (mg/dL) | | | | | |
|---|---|---|---|---|---|---|
| | 0 | 3.9 | 7.8 | 11.8 | 15.7 | 19.6 |
| Specimen No. 2 | 114 | 127 | 120 | 126 | 119 | 132 |
| To 0 (mg/dL) | - | 111% | 105% | 111% | 105% | 116% |

In the case that the hemolyzed hemoglobin was at 5 concentrations of 104, 208, 312, 416, and 520 mg/dL, the HRG concentration of the specimen was 101 to 110% with respect to the HRG concentration for the case that the hemolyzed hemoglobin was at 0 mg/dL. (Table 11)

**[Table 11]**

| HRG Concentration (µg/mL) | Hemolytic hemoglobin (mg/dL) | | | | | |
|---|---|---|---|---|---|---|
| | 0 | 104 | 208 | 312 | 416 | 520 |
| Specimen No. 2 | 113 | 122 | 120 | 125 | 123 | 114 |
| To 0 (mg/dL) | - | 108% | 106% | 110% | 109% | 101% |

The HRG concentration of the specimen afforded at 5 concentrations of 340, 680, 1020, 1360, and 1700 FTU of chyle was 94 to 100% with respect to the HRG concentration at 0 FTU of chyle. (Table 12)

**[Table 12]**

| HRG Concentration (µg/mL) | Chyle (FTU) | | | | | |
|---|---|---|---|---|---|---|
| | 0 | 340 | 680 | 1020 | 1360 | 1700 |
| Specimen No. 2 | 111 | 111 | 104 | 104 | 108 | 109 |
| To 0 (FTU) | - | 100% | 94% | 94% | 97% | 99% |

This sandwich ELISA system was indicated that it was a quantification system having no affection by the interfering substance and could be used for HRG quantification using a general plasma specimen.

### Example 12: Comparison with normal method (Examination of alternative method 6)

To compare the sandwich ELISA system (alternative method) with the sandwich ELISA system (normal method), HRG values of plasma specimens prepared from 10 septic patients were measured. The coefficient of variation between measurements when the same specimen was measured three times in different days by double measurement was calculated to confirm reproducibility.[Sandwich ELISA system (normal method)]

### (1) Test conditions

### (1-1) Preparation of buffer

0.1% BSA-containing PBS, pH 7.4 (GIBCO)

### (1-2) Preparation of antibody diluent

0.1% BSA-containing PBS, pH 7.4 (GIBCO)

### (1-3) Preparation of washing solution

0.05% Tween 20 in PBS, pH 7.4 (Takara)

### (1-4) Preparation of capture antibody

The purified anti-HRG antibody (75-2D) was diluted with a 0.2 M carbonate buffer to afford a capture antibody solution (10 pg/mL).

### (1-5) Preparation of detection antibody

The purified anti-HRG antibody (69-1A) was horseradish peroxidase (HRP) labeled followed by dilution with an antibody diluent to afford a detector antibody solution (75 ng/mL).

### (1-6) Preparation of standard substance for drawing calibration curve (human purified HRG protein)

396 pL of 0.1% BSA-containing PBS was mixed with 4.0 pL of purified HRG (1,000 pg/mL) to afford a standard substance solution for drawing calibration curve (10 pg/mL). The standard substance solution for drawing calibration curve (10 pg/mL) was diluted 100 times with 0.1% BSA-containing PBS, and the upper limit of the calibration curve was set to 100 ng/mL. The standard substance solution for calibration curve (100 ng/mL) was further diluted twice to afford a dilution series (50 ng/mL, 25 ng/mL, 12.5 ng/mL, 6.25 ng/mL, 3.125 ng/mL, 1.56 ng/mL, and 0 ng/mL).(1-7) Preparation of biological sample

Blood was collected from 10 septic patients to prepare plasma specimens (10 specimens). Each plasma specimen was diluted 5,000-fold with 0.1% BSA-containing PBS to be used for measurement.

### (2) Test method

The capture antibody solution was dispensed into 96-well Maxisorp plates (NUNC) at 100 pL per well followed by incubation at room temperature for 2 hours.

The plates were washed twice with 300 µL of washing solution per well (0.05%Tween 20-containing PBS). A blocking solution (1% BSA-containing PBS) was dispensed at 150 µL per well followed by incubation at room temperature for 2 hours. After removal of the blocking solution, the plates were washed twice with 300 µL of washing solution per well followed by dispensing the standard substance solution for calibration curve diluted in 7 steps and the biological samples (10 specimens) at 100 µL per well (n=2), and the plates were incubated at 4°C overnight.

After washing the plates four times with 300 pL of washing solution per well, the detector antibody solution was dispensed at 100 pL per well followed by incubating at room temperature for 2 hours. After washing the plates four times with the washing solution as described above, a chromogenic substrate solution (TMB (3,3',5,5'-tetramethylbenzidine)) was dispensed at 100 pL per well followed by incubating at room temperature for 10 minutes, then a reaction stop solution 0.5 M H₂SO₄ was added at 50 pL/well for measuring the absorbance at 450 nm. The measured absorbance value with respect to the HRG concentration of the standard substance were plotted to prepare calibration curves.

### [Sandwich ELISA system (alternative method)]

### (1) Test conditions

For the preparation of the buffer solution, the antibody diluent, the washing solution, the capture and the detector antibody, the test was performed using the same practices as those described in Example 7. For the preparation of the standard substance for calibration curve (human purified HRG protein) and the antibody-added buffer, the test was performed using the same practices as in Example 10.

### (1-1) Preparation of biological sample

Blood was collected from 10 septic patients to prepare plasma specimens (10 specimens). Each plasma specimen was diluted 101-fold with the buffer to be used for measurement.

### (2) Test method

The capture antibody solution was dispensed at 150 pL per well into 96-well Maxisorp plates (ThermoFisher, NUNC) followed by incubating at room temperature for 2 hours. The plates were washed twice with 300 pL of washing solution per well. A blocking solution (1% BSA-containing PBS) was dispensed at 300 pL per well. The plates were incubated at 4°C overnight or more. The prepared plates were stored at 4°C.

After removal of the blocking solution, the plates were washed twice with 300 pL of washing solution per well followed by dispensing the standard substance solution for calibration curve diluted in 5 steps at 25 pL per well (n=2). For the between-run reproducibility, biological samples (10 specimens) were dispensed at 25 µL per well (n=2) on each day.

The antibody-added buffer (7 pg/mL) was dispensed at 100 pL per well to each well to which the standard substance solution for calibration curve and the biological samples were added.

The plates were incubated at room temperature for 2 hours. After washing the plates three times with 300 pL of washing solution per well, the solution of detector antibody was dispensed at 100 µL per well followed by incubating at room temperature for 1 hour. After washing the plates three times with the washing solution as described above, a chromogenic substrate solution (TMB (3,3',5,5'-tetramethylbenzidine)) was dispensed at 100 pL per well followed by incubating at room temperature for 15 minutes, then a reaction stop solution 0.5 M H₂SO₄ was added at 50 µL/well for measuring the absorbance at 450 nm. The measured absorbance value with respect to the HRG concentration of the standard substance were plotted to give calibration curves.

### (3) Results

As a result of examining the reproducibility of the measurement system using the biological samples (10 specimens), the coefficient of variation of between-run reproducibility of the sandwich ELISA system (normal method) was 6.1 to 25.3% with 13.1% on average (Table 13), and the coefficient of variation of between-run reproducibility of the sandwich ELISA system (alternative method) was 3.5 to 11.3% with 5.8% on average (Table 14). This comes to conclusion that the sandwich ELISA system (alternative method) is an HRG quantification system having particularly high reproducibility.

**[Table 13]**

| Sandwich ELISA system (normal method) | | | Specimen Number | | | | | | | | | | |
|---|---|---|---|---|---|---|---|---|---|---|---|---|---|
| | | | HRG-1 | HRG-2 | HRG-3 | HRG-4 | HRG-5 | HRG-6 | HRG-7 | HRG-8 | HRG-9 | HRG-10 | |
| HRG Concentration (µg/mL) | Number of measurements | 1 | 83.1 | 162.8 | 108.7 | 146.1 | 76.3 | 131.6 | 65.9 | 80.6 | 42.4 | 74.4 | |
| | | 2 | 84.5 | 146.3 | 98.2 | 109.5 | 49.4 | 107.5 | 43.0 | 73.4 | 37.7 | 59.8 | |
| | | 3 | 99.1 | 193.5 | 113.5 | 104.9 | 51.6 | 130.5 | 55.5 | 82.4 | 42.6 | 67.4 | |
| Average | | | 88.9 | 167.5 | 106.8 | 120.2 | 59.1 | 123.2 | 54.8 | 78.8 | 40.9 | 67.2 | |
| Standard deviation | | | 8.9 | 23.9 | 7.9 | 22.6 | 14.9 | 13.6 | 11.5 | 4.8 | 2.8 | 7.3 | Average |
| CV% | | | 10.0% | 14.3% | 7.4% | 18.8% | 25.3% | 11.1% | 20.9% | 6.1% | 6.8% | 10.9% | 13.1 % |

**[Table 14]**

| Sandwich ELISA system (alternative method) | | | Specimen Number | | | | | | | | | | |
|---|---|---|---|---|---|---|---|---|---|---|---|---|---|
| | | | HRG-1 | HRG-2 | HRG-3 | HRG-4 | HRG-5 | HRG-6 | HRG-7 | HRG-8 | HRG-9 | HRG-10 | |
| HRG Concentration (µg/mL) | Number of measurements | 1 | 57.5 | 99.5 | 73.9 | 83.1 | 39.1 | 66.9 | 34.3 | 54.2 | 29.3 | 55.5 | |
| | | 2 | 54.8 | 102.1 | 70.0 | 78.1 | 37.2 | 65.9 | 33.2 | 50.8 | 34.2 | 51.2 | |
| | | 3 | 53.7 | 94.9 | 69.0 | 72.3 | 34.1 | 71.2 | 31.6 | 47.2 | 27.6 | 48.2 | |
| Average | | | 55.4 | 98.8 | 71.0 | 77.8 | 36.8 | 68.0 | 33.0 | 50.7 | 30.4 | 51.6 | |
| Standard deviation | | | 1.9 | 3.6 | 2.6 | 5.4 | 2.5 | 2.8 | 1.4 | 3.5 | 3.4 | 3.7 | Average |
| **CV%** | | | **3.5%** | **3.7%** | **3.6%** | **6.9%** | **6.8%** | **4.2%** | **4.2%** | **6.9%** | **11.3%** | **7.1%** | **5.8%** |

### Example 13: Comparison with commercial product (Examination of alternative method 7)

To compare the sandwich ELISA system (alternative method) with a commercially available HRG ELISA kit, HRG values of plasma specimens prepared from 10 septic patients were measured.

### [Commercial product]

### (1) Test conditions

[RayBio (registered trademark) Human HPRG ELISA Kit Catalog #: ELH-HPRG] was used as a commercial product.

### (2) Test method

The measurement was performed under the direction of the package insert. [Sandwich ELISA system (alternative method)]

### (1) Test conditions

For the preparation of the buffer solution, the antibody diluent, the washing solution, the capture and detector antibody, the test was performed using the same practices as those described in Example 7. For the preparation of the standard substance for calibration curve (human purified HRG protein) and the antibody-added buffer, the test was performed using the same conditions as in Example 10.

### (1-1) Preparation of biological sample

Blood was collected from 10 septic patients to prepare plasma specimens (10 specimens). Each plasma specimen was diluted 101-fold with the buffer to be used for measurement.

### (2) Test method

The capture antibody solution was dispensed at 150 pL per well into 96-well Maxisorp plates (ThermoFisher, NUNC) followed by incubating at room temperature for 2 hours. The plates were washed twice with 300 pL of washing solution per well. A blocking solution (1% BSA-containing PBS) was dispensed at 300 pL per well. The plates were incubated at 4°C overnight or more. The prepared plates were stored at 4°C.

After removal of the blocking solution, the plates were washed twice with 300 pL of washing solution per well followed by dispensing the standard substance solution for calibration curve diluted in 5 steps and the biological samples (10 specimens) at 25 pL per well (n=2).

The standard substance solution for calibration curve and the biological samples were added to each well followed by dispensing the antibody-added buffer (7 pg/mL) at 100 pL per well.

The plates were incubated at room temperature for 2 hours. After washing the plates three times with 300 µL of washing solution per well, the solution of detection antibody was dispensed at 100 pL per well followed by incubating at room temperature for 1 hour. After washing the plates three times with the washing solution as described above, a chromogenic substrate solution (TMB (3,3',5,5'-tetramethylbenzidine)) was dispensed at 100 pL per well followed by incubating at room temperature for 15 minutes, then a reaction stop solution 0.5 M H₂SO₄ was added at 50 pL/well for measuring the absorbance at 450 nm. The measured absorbance values with respect to the HRG concentration of the standard substance were plotted to give calibration curves.

### (3) Results

In the commercial product, it was come out that the HRG concentration in the plasma specimens was 30 to 79%, and 56.9% on average, which was measured lower than that in the sandwich ELISA system (alternative method). (Table 15) This suggested that there are possibly more HRGs in the specimens that cannot be detected by the commercial product. On the other hand, it was suggested that the sandwich ELISA system (alternative method) can measure HRG in the specimens that cannot be detected by the commercial product. Accordingly, it was indicated that the sandwich ELISA system (alternative method) can more accurately measure HRG in the specimens as compared with the commercial product.

**[Table 15]**

| | | Specimen Number | | | | | | | | | | |
|---|---|---|---|---|---|---|---|---|---|---|---|---|
| | | HRG-1 | HRG-2 | HRG-3 | HRG-4 | HRG-5 | HRG-6 | HRG-7 | HRG-8 | HRG-9 | HRG-10 | |
| HRG Concentration (µg/mL) | Sandwich ELISA system (alternative method) | 57.5 | 99.5 | 73.9 | 83.1 | 39.1 | 66.9 | 34.3 | 54.2 | 29.3 | 55.5 | |
| | Commercial product | 25.9 | 66.8 | 49.1 | 57.7 | 31.0 | 41.7 | 21.5 | 23.1 | 12.8 | 16.6 | Average |
| Commercial product /Sandwich ELISA system (alternative method) | | **45%** | **67%** | **66%** | **69%** | **79%** | **62%** | **63%** | **43%** | **44%** | **30%** | **56.9%** |

### [INDUSTRIAL APPLICABILITY]

The monoclonal antibody against HRG of the present invention, a set thereof and a method for measuring HRG using the same are useful for measurement in a test sample.

## Claims

1. A monoclonal antibody or antibody fragment thereof against HRG, comprising a heavy chain variable region and a light chain variable region according to any one of the following 1) to 6):
1) a heavy chain variable region comprising a CDR1 consisting of an amino acid sequence in which one to two amino acids may be deleted, substituted, inserted and/or added in the amino acid sequence of SEQ ID NO: 41,
a CDR2 consisting of an amino acid sequence in which one to two amino acids may be deleted, substituted, inserted and/or added in the amino acid sequence of SEQ ID NO: 42, and
a CDR3 consisting of an amino acid sequence in which one to two amino acids may be deleted, substituted, inserted and/or added in the amino acid sequence of SEQ ID NO: 43,
and
a light chain variable region comprising a CDR1 consisting of an amino acid sequence in which one to two amino acids may be deleted, substituted, inserted and/or added in the amino acid sequence of SEQ ID NO: 7,
a CDR2 consisting of an amino acid sequence in which one to two amino acids may be deleted, substituted, inserted and/or added in the amino acid sequence of SEQ ID NO: 45, and
a CDR3 consisting of an amino acid sequence in which one to two amino acids may be deleted, substituted, inserted and/or added in the amino acid sequence of SEQ ID NO: 9,
2) a heavy chain variable region comprising a CDR1 consisting of an amino acid sequence in which one to two amino acids may be deleted, substituted, inserted and/or added in the amino acid sequence of SEQ ID NO: 33,
a CDR2 consisting of an amino acid sequence in which one to two amino acids may be deleted, substituted, inserted and/or added in the amino acid sequence of SEQ ID NO: 34, and
a CDR3 consisting of an amino acid sequence in which one to two amino acids may be deleted, substituted, inserted and/or added in the amino acid sequence of SEQ ID NO: 35,
and
a light chain variable region comprising a CDR1 consisting of an amino acid sequence in which one to two amino acids may be deleted, substituted, inserted and/or added in the amino acid sequence of SEQ ID NO: 37,
a CDR2 consisting of an amino acid sequence in which one to two amino acids may be deleted, substituted, inserted and/or added in the amino acid sequence of SEQ ID NO: 38, and
a CDR3 consisting of an amino acid sequence in which one to two amino acids may be deleted, substituted, inserted and/or added in the amino acid sequence of SEQ ID NO: 39,
3) a heavy chain variable region comprising a CDR1 consisting of an amino acid sequence in which one to two amino acids may be deleted, substituted, inserted and/or added in the amino acid sequence of SEQ ID NO: 3,
a CDR2 consisting of an amino acid sequence in which one to two amino acids may be deleted, substituted, inserted and/or added in the amino acid sequence of SEQ ID NO: 4, and
a CDR3 consisting of an amino acid sequence in which one to two amino acids may be deleted, substituted, inserted and/or added in the amino acid sequence of SEQ ID NO: 5,
and
a light chain variable region comprising a CDR1 consisting of an amino acid sequence in which one to two amino acids may be deleted, substituted, inserted and/or added in the amino acid sequence of SEQ ID NO: 7,
a CDR2 consisting of an amino acid sequence in which one to two amino acids may be deleted, substituted, inserted and/or added in the amino acid sequence of SEQ ID NO: 8, and
a CDR3 consisting of an amino acid sequence in which one to two amino acids may be deleted, substituted, inserted and/or added in the amino acid sequence of SEQ ID NO: 9,
4) a heavy chain variable region comprising a CDR1 consisting of an amino acid sequence in which one to two amino acids may be deleted, substituted, inserted and/or added in the amino acid sequence of SEQ ID NO: 11,
a CDR2 consisting of an amino acid sequence in which one to two amino acids may be deleted, substituted, inserted and/or added in the amino acid sequence of SEQ ID NO: 12, and
a CDR3 consisting of an amino acid sequence in which one to two amino acids may be deleted, substituted, inserted and/or added in the amino acid sequence of SEQ ID NO: 13,
and
a light chain variable region comprising a CDR1 consisting of an amino acid sequence in which one to two amino acids may be deleted, substituted, inserted and/or added in the amino acid sequence of SEQ ID NO: 15,
a CDR2 consisting of an amino acid sequence in which one to two amino acids may be deleted, substituted, inserted and/or added in the amino acid sequence of SEQ ID NO: 16, and
a CDR3 consisting of an amino acid sequence in which one to two amino acids may be deleted, substituted, inserted and/or added in the amino acid sequence of SEQ ID NO: 17, and
5) a heavy chain variable region comprising a CDR1 consisting of an amino acid sequence in which one to two amino acids may be deleted, substituted, inserted and/or added in the amino acid sequence of SEQ ID NO: 21,
a CDR2 consisting of an amino acid sequence in which one to two amino acids may be deleted, substituted, inserted and/or added in the amino acid sequence of SEQ ID NO: 22, and
a CDR3 consisting of an amino acid sequence in which one to two amino acids may be deleted, substituted, inserted and/or added in the amino acid sequence of SEQ ID NO: 23,
and
a light chain variable region comprising a CDR1 consisting of an amino acid sequence in which one to two amino acids may be deleted, substituted, inserted and/or added in the amino acid sequence of SEQ ID NO: 25,
a CDR2 consisting of an amino acid sequence in which one to two amino acids may be deleted, substituted, inserted and/or added in the amino acid sequence of SEQ ID NO: 26, and
a CDR3 consisting of an amino acid sequence in which one to two amino acids may be deleted, substituted, inserted and/or added in the amino acid sequence of SEQ ID NO: 27,
6) a heavy chain variable region comprising a CDR1 consisting of an amino acid sequence in which one to two amino acids may be deleted, substituted, inserted and/or added in the amino acid sequence of SEQ ID NO: 11,
a CDR2 consisting of an amino acid sequence in which one to two amino acids may be deleted, substituted, inserted and/or added in the amino acid sequence of SEQ ID NO: 29, and
a CDR3 consisting of an amino acid sequence in which one to two amino acids may be deleted, substituted, inserted and/or added in the amino acid sequence of SEQ ID NO: 13,
and
a light chain variable region comprising a CDR1 consisting of an amino acid sequence in which one to two amino acids may be deleted, substituted, inserted and/or added in the amino acid sequence of SEQ ID NO: 15,
a CDR2 consisting of an amino acid sequence in which one to two amino acids may be deleted, substituted, inserted and/or added in the amino acid sequence of SEQ ID NO: 31, and
a CDR3 consisting of an amino acid sequence in which one to two amino acids may be deleted, substituted, inserted and/or added in the amino acid sequence of SEQ ID NO: 17.

2. The monoclonal antibody or antibody fragment thereof according to claim 1, comprising a heavy chain variable region and a light chain variable region according to any one of the following 1) to 7):
1) a heavy chain variable region consisting of an amino acid sequence in which one to two amino acids may be deleted, substituted, inserted and/or added in the amino acid sequence of SEQ ID NO: 40, and
a light chain variable region consisting of an amino acid sequence in which one to two amino acids may be deleted, substituted, inserted and/or added in the amino acid sequence of SEQ ID NO: 44,
2) a heavy chain variable region consisting of an amino acid sequence in which one to two amino acids may be deleted, substituted, inserted and/or added in the amino acid sequence of SEQ ID NO: 32, and
a light chain variable region consisting of an amino acid sequence in which one to two amino acids may be deleted, substituted, inserted and/or added in the amino acid sequence of SEQ ID NO: 36,
3) a heavy chain variable region consisting of an amino acid sequence in which one to two amino acids may be deleted, substituted, inserted and/or added in the amino acid sequence of SEQ ID NO: 2, and
a light chain variable region consisting of an amino acid sequence in which one to two amino acids may be deleted, substituted, inserted and/or added in the amino acid sequence of SEQ ID NO: 6,
4) a heavy chain variable region consisting of an amino acid sequence in which one to two amino acids may be deleted, substituted, inserted and/or added in the amino acid sequence of SEQ ID NO: 10, and
a light chain variable region consisting of an amino acid sequence in which one to two amino acids may be deleted, substituted, inserted and/or added in the amino acid sequence of SEQ ID NO: 14,
5) a heavy chain variable region consisting of an amino acid sequence in which one to two amino acids may be deleted, substituted, inserted and/or added in the amino acid sequence of SEQ ID NO: 18, and
a light chain variable region consisting of an amino acid sequence in which one to two amino acids may be deleted, substituted, inserted and/or added in the amino acid sequence of SEQ ID NO: 19,
6) a heavy chain variable region consisting of an amino acid sequence in which one to two amino acids may be deleted, substituted, inserted and/or added in the amino acid sequence of SEQ ID NO: 20, and
a light chain variable region consisting of an amino acid sequence in which one to two amino acids may be deleted, substituted, inserted and/or added in the amino acid sequence of SEQ ID NO: 24,
7) a heavy chain variable region consisting of an amino acid sequence in which one to two amino acids may be deleted, substituted, inserted and/or added in the amino acid sequence of SEQ ID NO: 28, and
a light chain variable region consisting of an amino acid sequence in which one to two amino acids may be deleted, substituted, inserted and/or added in the amino acid sequence of SEQ ID NO: 30.

3. A set of monoclonal antibodies or antibody fragments thereof against HRG, for use in measurement of HRG in a test sample, which is selected from the following 1) or 2):
1) a capture antibody is a monoclonal antibody or antibody fragment thereof against HRG, comprising:
a heavy chain variable region comprising a CDR1 consisting of an amino acid sequence in which one to two amino acids may be deleted, substituted, inserted and/or added in the amino acid sequence of SEQ ID NO: 41,
a CDR2 consisting of an amino acid sequence in which one to two amino acids may be deleted, substituted, inserted and/or added in the amino acid sequence of SEQ ID NO: 42, and
a CDR3 consisting of an amino acid sequence in which one to two amino acids may be deleted, substituted, inserted and/or added in the amino acid sequence of SEQ ID NO: 43,
and
a light chain variable region comprising a CDR1 consisting of an amino acid sequence in which one to two amino acids may be deleted, substituted, inserted and/or added in the amino acid sequence of SEQ ID NO: 7,
a CDR2 consisting of an amino acid sequence in which one to two amino acids may be deleted, substituted, inserted and/or added in the amino acid sequence of SEQ ID NO: 45, and
a CDR3 consisting of an amino acid sequence in which one to two amino acids may be deleted, substituted, inserted and/or added in the amino acid sequence of SEQ ID NO: 9, and
a detection antibody is a monoclonal antibody or antibody fragment thereof against HRG, comprising a heavy chain variable region and a light chain variable region according to any one of the following (i) to (iv):
(i) a heavy chain variable region comprising a CDR1 consisting of an amino acid sequence in which one to two amino acids may be deleted, substituted, inserted and/or added in the amino acid sequence of SEQ ID NO: 11,
a CDR2 consisting of an amino acid sequence in which one to two amino acids may be deleted, substituted, inserted and/or added in the amino acid sequence of SEQ ID NO: 12, and
a CDR3 consisting of an amino acid sequence in which one to two amino acids may be deleted, substituted, inserted and/or added in the amino acid sequence of SEQ ID NO: 13,
and
a light chain variable region comprising a CDR1 consisting of an amino acid sequence in which one to two amino acids may be deleted, substituted, inserted and/or added in the amino acid sequence of SEQ ID NO: 15,
a CDR2 consisting of an amino acid sequence in which one to two amino acids may be deleted, substituted, inserted and/or added in the amino acid sequence of SEQ ID NO: 16, and
a CDR3 consisting of an amino acid sequence in which one to two amino acids may be deleted, substituted, inserted and/or added in the amino acid sequence of SEQ ID NO: 17,
(ii) a heavy chain variable region comprising a CDR1 consisting of an amino acid sequence in which one to two amino acids may be deleted, substituted, inserted and/or added in the amino acid sequence of SEQ ID NO: 21,
a CDR2 consisting of an amino acid sequence in which one to two amino acids may be deleted, substituted, inserted and/or added in the amino acid sequence of SEQ ID NO: 22, and
a CDR3 consisting of an amino acid sequence in which one to two amino acids may be deleted, substituted, inserted and/or added in the amino acid sequence of SEQ ID NO: 23,
and
a light chain variable region comprising a CDR1 consisting of an amino acid sequence in which one to two amino acids may be deleted, substituted, inserted and/or added in the amino acid sequence of SEQ ID NO: 25,
a CDR2 consisting of an amino acid sequence in which one to two amino acids may be deleted, substituted, inserted and/or added in the amino acid sequence of SEQ ID NO: 26, and
a CDR3 consisting of an amino acid sequence in which one to two amino acids may be deleted, substituted, inserted and/or added in the amino acid sequence of SEQ ID NO: 27,
(iii) a heavy chain variable region comprising a CDR1 consisting of an amino acid sequence in which one to two amino acids may be deleted, substituted, inserted and/or added in the amino acid sequence of SEQ ID NO: 11,
a CDR2 consisting of an amino acid sequence in which one to two amino acids may be deleted, substituted, inserted and/or added in the amino acid sequence of SEQ ID NO: 29, and
a CDR3 consisting of an amino acid sequence in which one to two amino acids may be deleted, substituted, inserted and/or added in the amino acid sequence of SEQ ID NO: 13,
and
a light chain variable region comprising a CDR1 consisting of an amino acid sequence in which one to two amino acids may be deleted, substituted, inserted and/or added in the amino acid sequence of SEQ ID NO: 15,
a CDR2 consisting of an amino acid sequence in which one to two amino acids may be deleted, substituted, inserted and/or added in the amino acid sequence of SEQ ID NO: 31, and
a CDR3 consisting of an amino acid sequence in which one to two amino acids may be deleted, substituted, inserted and/or added in the amino acid sequence of SEQ ID NO: 17,
(iv) a monoclonal antibody or antibody fragment thereof against HRG, comprising a heavy chain variable region comprising a CDR1 consisting of an amino acid sequence in which one to two amino acids may be deleted, substituted, inserted and/or added in the amino acid sequence of SEQ ID NO: 33,
a CDR2 consisting of an amino acid sequence in which one to two amino acids may be deleted, substituted, inserted and/or added in the amino acid sequence of SEQ ID NO: 34, and
a CDR3 consisting of an amino acid sequence in which one to two amino acids may be deleted, substituted, inserted and/or added in the amino acid sequence of SEQ ID NO: 35,
and
a light chain variable region comprising a CDR1 consisting of an amino acid sequence in which one to two amino acids may be deleted, substituted, inserted and/or added in the amino acid sequence of SEQ ID NO: 37,
a CDR2 consisting of an amino acid sequence in which one to two amino acids may be deleted, substituted, inserted and/or added in the amino acid sequence of SEQ ID NO: 38, and
a CDR3 consisting of an amino acid sequence in which one to two amino acids may be deleted, substituted, inserted and/or added in the amino acid sequence of SEQ ID NO: 39,
2) a capture antibody is a monoclonal antibody or antibody fragment thereof against HRG, comprising:
a heavy chain variable region comprising a CDR1 consisting of an amino acid sequence in which one to two amino acids may be deleted, substituted, inserted and/or added in the amino acid sequence of SEQ ID NO: 3,
a CDR2 consisting of an amino acid sequence in which one to two amino acids may be deleted, substituted, inserted and/or added in the amino acid sequence of SEQ ID NO: 4, and
a CDR3 consisting of an amino acid sequence in which one to two amino acids may be deleted, substituted, inserted and/or added in the amino acid sequence of SEQ ID NO: 5,
and
a light chain variable region comprising a CDR1 consisting of an amino acid sequence in which one to two amino acids may be deleted, substituted, inserted and/or added in the amino acid sequence of SEQ ID NO: 7,
a CDR2 consisting of an amino acid sequence in which one to two amino acids may be deleted, substituted, inserted and/or added in the amino acid sequence of SEQ ID NO: 8, and
a CDR3 consisting of an amino acid sequence in which one to two amino acids may be deleted, substituted, inserted and/or added in the amino acid sequence of SEQ ID NO: 9, and
a detection antibody is a monoclonal antibody or antibody fragment thereof against HRG, comprising a heavy chain variable region and a light chain variable region according to any one of the following (i) to (iii):
(i) a heavy chain variable region comprising a CDR1 consisting of an amino acid sequence in which one to two amino acids may be deleted, substituted, inserted and/or added in the amino acid sequence of SEQ ID NO: 11,
a CDR2 consisting of an amino acid sequence in which one to two amino acids may be deleted, substituted, inserted and/or added in the amino acid sequence of SEQ ID NO: 12, and
a CDR3 consisting of an amino acid sequence in which one to two amino acids may be deleted, substituted, inserted and/or added in the amino acid sequence of SEQ ID NO: 13,
and
a light chain variable region comprising a CDR1 consisting of an amino acid sequence in which one to two amino acids may be deleted, substituted, inserted and/or added in the amino acid sequence of SEQ ID NO: 15,
a CDR2 consisting of an amino acid sequence in which one to two amino acids may be deleted, substituted, inserted and/or added in the amino acid sequence of SEQ ID NO: 16, and
a CDR3 consisting of an amino acid sequence in which one to two amino acids may be deleted, substituted, inserted and/or added in the amino acid sequence of SEQ ID NO: 17,
(ii) a heavy chain variable region comprising a CDR1 consisting of an amino acid sequence in which one to two amino acids may be deleted, substituted, inserted and/or added in the amino acid sequence of SEQ ID NO: 11,
a CDR2 consisting of an amino acid sequence in which one to two amino acids may be deleted, substituted, inserted and/or added in the amino acid sequence of SEQ ID NO: 29, and
a CDR3 consisting of an amino acid sequence in which one to two amino acids may be deleted, substituted, inserted and/or added in the amino acid sequence of SEQ ID NO: 13,
and
a light chain variable region comprising a CDR1 consisting of an amino acid sequence in which one to two amino acids may be deleted, substituted, inserted and/or added in the amino acid sequence of SEQ ID NO: 15,
a CDR2 consisting of an amino acid sequence in which one to two amino acids may be deleted, substituted, inserted and/or added in the amino acid sequence of SEQ ID NO: 31, and
a CDR3 consisting of an amino acid sequence in which one to two amino acids may be deleted, substituted, inserted and/or added in the amino acid sequence of SEQ ID NO: 17,
(iii) a monoclonal antibody or antibody fragment thereof against HRG, comprising a heavy chain variable region comprising a CDR1 consisting of an amino acid sequence in which one to two amino acids may be deleted, substituted, inserted and/or added in the amino acid sequence of SEQ ID NO: 33,
a CDR2 consisting of an amino acid sequence in which one to two amino acids may be deleted, substituted, inserted and/or added in the amino acid sequence of SEQ ID NO: 34, and
a CDR3 consisting of an amino acid sequence in which one to two amino acids may be deleted, substituted, inserted and/or added in the amino acid sequence of SEQ ID NO: 35,
and
a light chain variable region comprising a CDR1 consisting of an amino acid sequence in which one to two amino acids may be deleted, substituted, inserted and/or added in the amino acid sequence of SEQ ID NO: 37,
a CDR2 consisting of an amino acid sequence in which one to two amino acids may be deleted, substituted, inserted and/or added in the amino acid sequence of SEQ ID NO: 38, and
a CDR3 consisting of an amino acid sequence in which one to two amino acids may be deleted, substituted, inserted and/or added in the amino acid sequence of SEQ ID NO: 39.

4. The set of monoclonal antibodies or antibody fragments thereof according to claim 3, which is selected from the following 1) or 2):
1) a capture antibody is a monoclonal antibody or antibody fragment thereof against HRG, comprising:
a heavy chain variable region consisting of an amino acid sequence in which one to two amino acids may be deleted, substituted, inserted and/or added in the amino acid sequence of SEQ ID NO: 40, and
a light chain variable region consisting of an amino acid sequence in which one to two amino acids may be deleted, substituted, inserted and/or added in the amino acid sequence of SEQ ID NO: 44, and
a detection antibody is a monoclonal antibody or antibody fragment thereof against HRG, comprising a heavy chain variable region and a light chain variable region according to any one of the following (i) to (v):
(i) a heavy chain variable region consisting of an amino acid sequence in which one to two amino acids may be deleted, substituted, inserted and/or added in the amino acid sequence of SEQ ID NO: 10, and
a light chain variable region consisting of an amino acid sequence in which one to two amino acids may be deleted, substituted, inserted and/or added in the amino acid sequence of SEQ ID NO: 14,
(ii) a heavy chain variable region consisting of an amino acid sequence in which one to two amino acids may be deleted, substituted, inserted and/or added in the amino acid sequence of SEQ ID NO: 18, and
a light chain variable region consisting of an amino acid sequence in which one to two amino acids may be deleted, substituted, inserted and/or added in the amino acid sequence of SEQ ID NO: 19,
(iii) a heavy chain variable region consisting of an amino acid sequence in which one to two amino acids may be deleted, substituted, inserted and/or added in the amino acid sequence of SEQ ID NO: 20, and
a light chain variable region consisting of an amino acid sequence in which one to two amino acids may be deleted, substituted, inserted and/or added in the amino acid sequence of SEQ ID NO: 24,
(iv) a monoclonal antibody or antibody fragment thereof against HRG, comprising a heavy chain variable region consisting of an amino acid sequence in which one to two amino acids may be deleted, substituted, inserted and/or added in the amino acid sequence of SEQ ID NO: 28, and
a light chain variable region consisting of an amino acid sequence in which one to two amino acids may be deleted, substituted, inserted and/or added in the amino acid sequence of SEQ ID NO: 30, and
(v) a heavy chain variable region consisting of an amino acid sequence in which one to two amino acids may be deleted, substituted, inserted and/or added in the amino acid sequence of SEQ ID NO: 32, and
a light chain variable region consisting of an amino acid sequence in which one to two amino acids may be deleted, substituted, inserted and/or added in the amino acid sequence of SEQ ID NO: 36,
2) a capture antibody is a monoclonal antibody or antibody fragment thereof against HRG, comprising:
a heavy chain variable region consisting of an amino acid sequence in which one to two amino acids may be deleted, substituted, inserted and/or added in the amino acid sequence of SEQ ID NO: 2, and
a light chain variable region consisting of an amino acid sequence in which one to two amino acids may be deleted, substituted, inserted and/or added in the amino acid sequence of SEQ ID NO: 6, and
a detection antibody is a monoclonal antibody or antibody fragment thereof against HRG, comprising a heavy chain variable region and a light chain variable region according to any one of the following (i) to (iv):
(i) a heavy chain variable region consisting of an amino acid sequence in which one to two amino acids may be deleted, substituted, inserted and/or added in the amino acid sequence of SEQ ID NO: 10, and
a light chain variable region consisting of an amino acid sequence in which one to two amino acids may be deleted, substituted, inserted and/or added in the amino acid sequence of SEQ ID NO: 14,
(ii) a heavy chain variable region consisting of an amino acid sequence in which one to two amino acids may be deleted, substituted, inserted and/or added in the amino acid sequence of SEQ ID NO: 18, and
a light chain variable region consisting of an amino acid sequence in which one to two amino acids may be deleted, substituted, inserted and/or added in the amino acid sequence of SEQ ID NO: 19,
(iii) a heavy chain variable region consisting of an amino acid sequence in which one to two amino acids may be deleted, substituted, inserted and/or added in the amino acid sequence of SEQ ID NO: 28, and
a light chain variable region consisting of an amino acid sequence in which one to two amino acids may be deleted, substituted, inserted and/or added in the amino acid sequence of SEQ ID NO: 30, and
(iv) a heavy chain variable region consisting of an amino acid sequence in which one to two amino acids may be deleted, substituted, inserted and/or added in the amino acid sequence of SEQ ID NO: 32, and
a light chain variable region consisting of an amino acid sequence in which one to two amino acids may be deleted, substituted, inserted and/or added in the amino acid sequence of SEQ ID NO: 36.

5. A kit for detecting or quantifying HRG in a test sample, comprising the monoclonal antibody or antibody fragment thereof according to claim 1 or 2 or the set of monoclonal antibodies or antibody fragments thereof according to claim 3 or 4.

6. The kit according to claim 5, wherein the detection or quantification of HRG in a test sample is performed by any immunoassay selected from enzyme immunoassay (ELISA or EIA), fluorescence immunoassay (FIA), radioimmunoassay (RIA), and chemiluminescence immunoassay.

7. The kit according to claim 6, wherein the immunoassay is a sandwich ELISA method.

8. The kit according to claim 7, wherein the test sample is blood.

9. The kit according to claim 8, for detection of sepsis.

10. A method for measuring HRG in a test sample using a capture antibody and a detection antibody, the method comprising:
forming a sandwich immune complex containing a capture antibody, HRG, and a detection antibody,
wherein the capture antibody is a monoclonal antibody or antibody fragment thereof against HRG, comprising a heavy chain variable region comprising a CDR1 consisting of an amino acid sequence in which one to two amino acids may be deleted, substituted, inserted and/or added in the amino acid sequence of SEQ ID NO: 41,
a CDR2 consisting of an amino acid sequence in which one to two amino acids may be deleted, substituted, inserted and/or added in the amino acid sequence of SEQ ID NO: 42, and
a CDR3 consisting of an amino acid sequence in which one to two amino acids may be deleted, substituted, inserted and/or added in the amino acid sequence of SEQ ID NO: 43,
and
a light chain variable region comprising a CDR1 consisting of an amino acid sequence in which one to two amino acids may be deleted, substituted, inserted and/or added in the amino acid sequence of SEQ ID NO: 7,
a CDR2 consisting of an amino acid sequence in which one to two amino acids may be deleted, substituted, inserted and/or added in the amino acid sequence of SEQ ID NO: 45, and
a CDR3 consisting of an amino acid sequence in which one to two amino acids may be deleted, substituted, inserted and/or added in the amino acid sequence of SEQ ID NO: 9, and
the detection antibody is a monoclonal antibody or antibody fragment thereof against HRG, comprising a heavy chain variable region comprising a CDR1 consisting of an amino acid sequence in which one to two amino acids may be deleted, substituted, inserted and/or added in the amino acid sequence of SEQ ID NO: 33,
a CDR2 consisting of an amino acid sequence in which one to two amino acids may be deleted, substituted, inserted and/or added in the amino acid sequence of SEQ ID NO: 34, and
a CDR3 consisting of an amino acid sequence in which one to two amino acids may be deleted, substituted, inserted and/or added in the amino acid sequence of SEQ ID NO: 35,
and
a light chain variable region comprising a CDR1 consisting of an amino acid sequence in which one to two amino acids may be deleted, substituted, inserted and/or added in the amino acid sequence of SEQ ID NO: 37,
a CDR2 consisting of an amino acid sequence in which one to two amino acids may be deleted, substituted, inserted and/or added in the amino acid sequence of SEQ ID NO: 38, and
a CDR3 consisting of an amino acid sequence in which one to two amino acids may be deleted, substituted, inserted and/or added in the amino acid sequence of SEQ ID NO: 39.

11. The measurement method according to claim 10, wherein
the capture antibody is a monoclonal antibody or antibody fragment thereof against HRG, comprising a heavy chain variable region consisting of an amino acid sequence in which one to two amino acids may be deleted, substituted, inserted and/or added in the amino acid sequence of SEQ ID NO: 40, and
a light chain variable region consisting of an amino acid sequence in which one to two amino acids may be deleted, substituted, inserted and/or added in the amino acid sequence of SEQ ID NO: 44, and
the detection antibody is a monoclonal antibody or antibody fragment thereof against HRG, comprising a heavy chain variable region consisting of an amino acid sequence in which one to two amino acids may be deleted, substituted, inserted and/or added in the amino acid sequence of SEQ ID NO: 32, and
a light chain variable region consisting of an amino acid sequence in which one to two amino acids may be deleted, substituted, inserted and/or added in the amino acid sequence of SEQ ID NO: 36.

12. The measurement method according to claim 10 or 11, for detection of sepsis.

13. A method for measuring HRG in a test sample using a set of monoclonal antibodies or antibody fragments thereof against HRG comprising a combination of a capture antibody and a detection antibody, the method comprising:
(a) reacting a test sample with a capture antibody immobilized on a solid phase in a reaction solution containing an antibody of the same type as the capture antibody or antibody fragment thereof to produce an immune complex containing the capture antibody immobilized on the solid phase and HRG; and
(b) measuring the immune complex produced in the (a) using a detection antibody.

14. The measurement method according to claim 13, wherein the set is a set of monoclonal antibodies or antibody fragments thereof according to claim 3 or 4.
